# EUROPEAN PATENT SPECIFICATION

(11) **EP 4 382 517 B1**
(45) Date of publication and mention of the grant of the patent: **28.01.2026**
(21) Application number: 23843406.2
(22) Date of filing: 20.07.2023
(51) Int. Cl.: C07D 319/12, C08J 11/12, C08J 11/16, C07C 7/148, C07C 11/16, C08J 11/10, C07C 51/347, C07C 51/487, B09B 3/40, B09B 3/70, C07C 1/24

(54) **BUTADIENE PREPARATION METHOD**
BUTADIENHERSTELLUNGSVERFAHREN
PROCÉDÉ DE PRÉPARATION DE BUTADIÈNE

(30) Priority: 20.07.2022 KR 20220089692; 20.07.2022 KR 20220089693; 20.07.2022 KR 20220089694; 26.09.2022 KR 20220121582; 05.10.2022 KR 20220127102; 19.07.2023 KR 20230094098
(43) Date of publication of application: 12.06.2024
(73) Proprietor: LG Chem, Ltd., Yeongdeungpo-gu Seoul 07336 (KR)
(72) Inventor: KIM, Si Min, Daejeon 34122 (KR); KANG, Donggyun, Daejeon 34122 (KR); JUNG, Woochul, Daejeon 34122 (KR)
(74) Representative: Goddar, Heinz J.
(86) International application number: PCT/KR2023/010475
(87) International publication number: WO 2024/019560

(56) References cited:
- KR-A- 20150 032 579
- US-A1- 2012 315 681
- VIJAKAKUMAR C T ET AL: "THERMAL AND PYROLYSIS STUDIES OF COPOLYESTERS", JOURNAL OF POLYMER SCIENCE, POLYMER LETTERS EDITION, JOHN WILEY AND SONS. NEW YORK, US, vol. 20, no. 9, 1 September 1982 (1982-09-01), pages 2715 - 2725, XP001109662
- CHIU S J ET AL: "A comparative study on thermal and catalytic degradation of polybutylene terephthalate", JOURNAL OF ANALYTICAL AND APPLIED PYROLYSIS, ELSEVIER BV, NL, vol. 86, no. 1, 1 September 2009 (2009-09-01), pages 22 - 27, XP026445589, ISSN: 0165-2370, [retrieved on 20090324]
- AMANDINE VIRETTO ET AL: "Thermal degradation of polyesters filled with magnesium dihydroxide and magnesium oxide", FIRE AND MATERIALS, NEW YORK, NY, US, vol. 40, no. 3, 27 February 2015 (2015-02-27), pages 445 - 463, XP071613570, ISSN: 0308-0501, DOI: 10.1002/FAM.2299
- XIANG SHENG ET AL: "Evaluation of PLA content in PLA/PBAT blends using TGA", POLYMER TESTING, vol. 81, 1 January 2020 (2020-01-01), AMSTERDAM, NL, pages 106211, XP093272945, ISSN: 0142-9418, DOI: 10.1016/j.polymertesting.2019.106211
- ARIFFIN H ET AL: "Highly selective transformation of poly[(R)-3-hydroxybutyric acid] into trans-crotonic acid by catalytic thermal degradation", POLYMER DEGRADATION AND STABILITY, BARKING, GB, vol. 95, no. 8, 1 August 2010 (2010-08-01), pages 1375 - 1381, XP027122908, ISSN: 0141-3910, [retrieved on 20100128]
- FENG LIDONG ET AL: "Pyrolysis mechanism of Poly(lactic acid) for giving lactide under the catalysis of tin", POLYMER DEGRADATION AND STABILITY, vol. 157, 1 November 2018 (2018-11-01), GB, pages 212 - 223, XP093130595, ISSN: 0141-3910, DOI: 10.1016/j.polymdegradstab.2018.10.008
- YOSHIOKA, T. ; GRAUSE, G. ; OTANI, S. ; OKUWAKI, A.: "Selective production of benzene and naphthalene from poly(butylene terephthalate) and poly(ethylene naphthalene-2,6-dicarboxylate) by pyrolysis in the presence of calcium hydroxide", POLYMER DEGRADATION AND STABILITY, BARKING, GB, vol. 91, no. 5, 1 May 2006 (2006-05-01), GB , pages 1002 - 1009, XP027949504, ISSN: 0141-3910
- FENG LIDONG, FENG SONGYANG, BIAN XINCHAO, LI GAO, CHEN XUESI: "Pyrolysis mechanism of Poly(lactic acid) for giving lactide under the catalysis of tin", POLYMER DEGRADATION AND STABILITY, BARKING, GB, vol. 157, 1 November 2018 (2018-11-01), GB , pages 212 - 223, XP093130595, ISSN: 0141-3910, DOI: 10.1016/j.polymdegradstab.2018.10.008
- SIGNORI, F. ; COLTELLI, M.B. ; BRONCO, S.: "Thermal degradation of poly(lactic acid) (PLA) and poly(butylene adipate-co-terephthalate) (PBAT) and their blends upon melt processing", POLYMER DEGRADATION AND STABILITY, BARKING, GB, vol. 94, no. 1, 1 January 2009 (2009-01-01), GB , pages 74 - 82, XP025799264, ISSN: 0141-3910, DOI: 10.1016/j.polymdegradstab.2008.10.004
- MARINHO VITHÓRIA A.D.; PEREIRA CAMILA A.B.; VITORINO MARIA B.C.; SILVA ALINE S.; CARVALHO LAURA H.; CANEDO EDUARDO L.: "Degradation and recovery in poly(butylene adipate-co-terephthalate)/ thermoplastic starch blends", POLYMER TESTING, ELSEVIER, AMSTERDAM, NL, vol. 58, 26 December 2016 (2016-12-26), AMSTERDAM, NL , pages 166 - 172, XP029922303, ISSN: 0142-9418, DOI: 10.1016/j.polymertesting.2016.12.028
- JALIL R., NIXON J. R.: "Biodegradable poly(lactic acid) and poly(lactide-co-glycolide) microcapsules: problems associated with preparative techniques and release properties.", JOURNAL OF MICROENCAPSULATION, vol. 07., no. 03., 1 July 1990 (1990-07-01), GB , pages 297 - 325., XP000142031, ISSN: 0265-2048

## Description

### FIELD OF THE INVENTION

The present invention relates to a method of manufacturing butadiene by thermally decomposing a polyester containing a repeating unit derived from 1,4-butanediol or a polymer blend containing the polyester.

### BACKGROUND OF THE INVENTION

Plastics are inexpensive and durable materials, which can be used to manufacture a variety of products that find use in a wide range of applications. As a consequence, the production of plastics has increased dramatically over the last decades. Moreover, more than 50% of these plastics are used for single-use disposable applications, such as packaging, agricultural films, disposable consumer items or for short-lived products that are discarded within a year of manufacture. Because of the durability of the polymers involved, substantial quantities of plastics are piling up in landfill sites and in natural habitats worldwide, generating increasing environmental problems. Even biodegradable plastics may persist for decades depending on local environmental factors, like levels of ultraviolet light exposure, temperature, presence of suitable microorganisms, etc.

Different solutions have been studied to reduce economic and environmental impacts correlated to the accumulation of plastic, from plastic degradation to plastic recycling.

As an example, polyethylene terephthalate (PET) is the most closed-loop recycled plastic (a system that processes and recycles wastes generated from the manufacturing process). PET wastes (mainly bottles) are collected, sorted, pressed into bales, crushed, washed, chopped into flakes, melted and extruded in pellets and offered for sale. However, such plastic recycling processes are adapted to plastic items containing only PET, and thus need prior extensive sorting.

Another potential process for recycling plastic consists of chemical recycling allowing recovering the chemical constituents of the polymer. The resulting monomers, after purification, may be used to re-manufacture plastic items. Thus, a need exists for chemical regeneration methods to recycle the polymers.

Journal of Polymer Science, Polymer Letters Edition, Volume 20, Number 9, pages 2715-2725, discloses a method wherein the random copolymer dimethyl terephthalate, ethylene glycol and butane-1,4-diol and the homopolymer poly(butylene terephthalate) have been subjected to degradation and pyrolysis, wherein the polymers are heated to 440°C, maintaining the temperature for 5 minutes.

Journal of Analytical and Applied Pyrolysis, Volume 86, Number 1, pages 22-27, discloses a thermal and catalytic degradation method of polybutylene terephthalate carried out at a temperature up to 400°C, with a formation of 1,3-butadiene, wherein the catalyst includes Sn₂(OAc).

Fire and Materials, Volume 40, Number 3, pages 445-463, discloses a method of thermal degradation of polybutylene terephthalate in the presence of MgO with the release of butadiene at 420°C.

Polymer Testing, Volume 81, page 106211, discloses a method wherein a blend of polylactide and poly(butylene adipate-co-terephthalate) at weight ratios from 5/95 to 95/5 incorporating MgO is thermally decomposed by heating the solvent-free blend at a heating rate of 2°C/minute up to 450-500°C.

Polymer Degradation and Stability, Volume 91, Number 5, pages 1002-1009, discloses the thermal degradation of polybutylene terephthalate in a fixed bed reactor in the presence of calcium hydroxide up to a temperature of 600°C to obtain 1,3-butadiene.

### SUMMARY OF THE INVENTION

### TECHNICAL PROBLEM

It is an object of the present invention to provide a method of manufacturing butadiene by thermally decomposing a polyester containing a repeating unit derived from 1,4-butanediol or a polymer blend containing the polyester.

### TECHNICAL SOLUTION

According to the present invention, there is provided a method of manufacturing butadiene comprising thermally decomposing a polyester containing a repeating unit derived from 1,4-butanediol or a polymer blend containing the polyester.

Now, a method of manufacturing butadiene according to specific embodiments of the invention will be described in more detail.

Unless particularly mentioned herein, the term "including" or "containing" refers to including some element (or component) without any limitation, and should not be construed as excluding addition of other elements (or components).

Further, if the steps constituting the manufacturing method described herein are specified as being sequential or continuous, or unless otherwise specified, one step and other steps constituting one manufacturing method shall not be construed as limited to the order described herein. Therefore, the order of the steps constituting the manufacturing method can be changed within a range that can be easily understood by a person skilled in the art, and in this case, the accompanying changes that are obvious to a person skilled in the art are not included in the scope of the present invention.

Further, unless otherwise stated herein, the weight average molecular weight can be measured using a gel permeation chromatography (GPC). Specifically, the polyester is dissolved in chloroform to a concentration of 2 mg/ml, then 20 µℓ of the dissolved solution is injected into GPC, and GPC analysis is carried out at 40°C. At this time, chloroform is used as the mobile phase for GPC, the flow rate is 1.0 mL/min, and the column used is two Agilent Mixed-B units connected in series. RI Detector is used as a detector. The values of Mw are obtained using a calibration curve formed using a polystyrene standard sample. Nine kinds of the polystyrene standard samples are used with the molecular weight of 2,000 g/mol, 10,000 g/mol, 30,000 g/mol, 70,000 g/mol, 200,000 g/mol, 700,000 g/mol, 2,000,000 g/mol, 4,000,000 g/mol, and 10,000,000 g/mol.

The terms "first", "second", "third", and so forth are used to describe diverse processes, and the terms are used only to discriminate one constituent element (process) from another constituent element (process).

In this specification, a polymer blend is produced by mechanically or chemically thoroughly mixing polymers produced by polymerization of monomers, and can be produced, for example, by compounding two or more polymers in a molten state.

According to the present invention, there is provided a method of manufacturing butadiene comprising thermally decomposing a polyester containing a repeating unit derived from 1,4-butanediol or a polymer blend containing the polyester to manufacture butadiene, wherein
the thermal decomposition is carried out under a tin catalyst,
the thermal decomposition is carried out at a temperature of at least 400°C,
the thermal decomposition is carried out under a solvent-free condition, and wherein the tin catalyst is at least one selected from the group consisting of tin(II) 2-ethylhexanoate, tin(II) 2-methylhexanoate, tin(II) 2-propylhexanoate, dioctyltin dilaurate, dihexyltin dilaurate, dibutyltin dilaurate, dipropyltin dilaurate, diethyltin dilaurate, dimetyltin dilaurate, dibutyltin bis(lauryl mercaptide), dimethyltin bis(lauryl mercaptide), diethyltin bis(lauryl mercaptide), dipropyltin bis(lauryl mercaptide), dihexyltin bis(lauryl mercaptide), dioctyltin bis(lauryl mercaptide), dimethyltin bis(isooctylmaleate), diethyltin bis(isooctylmaleate), dipropyltin bis(isooctylmaleate), dibutyltin bis(isooctylmaleate), dihexyltin bis(isooctylmaleate) and dioctyltin bis(isooctylmaleate),
the polyester containing a repeating unit derived from 1,4-butanediol is polybutylene adipate terephthalate(PBAT).

Further embodiments are disclosed in the dependent claims.

The present inventors have found that when polyester containing a repeating unit derived from 1,4-butanediol or a polymer blend containing the polyester is thermally decomposed, butadiene, and the like, which are recyclable monomers, can be recovered with high purity and high yield, respectively, and completed the present invention.

Further, since recyclable monomers are produced by thermally decomposing a polyester containing a repeating unit derived from 1,4-butanediol or a polymer blend containing the polyester, it is environmentally friendly and economical.

The method of manufacturing butadiene according to one embodiment may, before the step of thermally decomposing a polyester containing a repeating unit derived from 1,4-butanediol or a polymer blend containing the polyester to manufacture butadiene, further comprise melting the polyester containing a repeating unit derived from 1,4-butanediol or the polymer blend containing the polyester. That is, the polyester containing the repeating unit derived from 1,4-butanediol or the polymer blend containing the polyester can be melted before the thermal decomposition.

Before the thermal decomposition, volatile substances remaining due to melting and/or impurities flowing in the recycling can be removed. Further, due to the melting, the mobility of a polyester or a polymer blend containing the polyester increases, thus allowing it to be easily placed in a thermal decomposition reactor. Taking advantage of this point, the thermal decomposition can proceed continuously at a later time.

The melting may be carried out at a temperature of 150°C or more and 280°C or less. For example, the temperature during the melting may be 150°C or more, 160°C or more, 170°C or more, or 180°C or more, and 280°C or less, 270°C or less, 260°C or less, or 250°C or less. If the melting temperature is too low, the polyester containing a repeating unit derived from 1,4-butanediol or the polymer blend containing the polyester may not melt, and if the melting temperature is too high, the polyester containing a repeating unit derived from 1,4-butanediol or the polymer blend containing the polyester does not melt but undergoes thermal decomposition, which leads to a decrease in the recovery rate of monomers, or since there is no process to remove impurities before thermal decomposition, a lot of impurities may flow in, and bumping may occur due to a sudden temperature rise.

On the other hand, the melting may be carried out under a solvent-free condition. For example, if the melting is carried out under a solvent-free condition, the polyester containing a repeating unit derived from 1,4-butanediol or the polymer blend containing the polyester may be melted without being dissolved in a solvent. For example, no other solvent is placed in a reactor in addition to a polyester containing a repeating unit derived from 1,4-butanediol or a polymer blend containing the polyester, the temperature applied to the reactor may be directly transferred to the polyester containing a repeating unit derived from 1,4-butanediol or the polymer blend containing the polyester to melt it. When a solvent is placed in a reactor, impurities may be formed during the process of thermally decomposing the polyester containing a repeating unit derived from 1,4-butanediol or the polymer blend containing the polyester, and solvent removal steps and additional impurity removal steps are required, which may make the process complicate and require additional equipment. Further, side reactions may occur during the process of removing the solvent, which leads to a decrease in the recovery rate and purity of monomers. Further, there is a disadvantage that the economic efficiency is lowered due to the use of additional solvents.

In the method of manufacturing butadiene according to the present invention, thermal decomposition is carried out under a tin catalyst.

The tin catalyst is at least one selected from the group consisting of tin(II) 2-ethylhexanoate, tin(II) 2-methylhexanoate, tin(II) 2-propylhexanoate, dioctyltin dilaurate, dihexyltin dilaurate, dibutyltin dilaurate, dipropyltin dilaurate, diethyltin dilaurate, dimetyltin dilaurate, dibutyltin bis(lauryl mercaptide), dimethyltin bis(lauryl mercaptide), diethyltin bis(lauryl mercaptide), dipropyltin bis(lauryl mercaptide), dihexyltin bis(lauryl mercaptide), dioctyltin bis(lauryl mercaptide), dimethyltin bis(isooctylmaleate), diethyltin bis(isooctylmaleate), dipropyltin bis(isooctylmaleate), dibutyltin bis(isooctylmaleate), dihexyltin bis(isooctylmaleate) and dioctyltin bis(isooctylmaleate).

The tin catalyst can be used in an amount of 0.0001 parts by weight or more, 0.0010 parts by weight or more, 0.0100 parts by weight or more, or 0.1000 parts by weight or more, and 10 parts by weight or less, 7 parts by weight or less, 5 parts by weight or less, 3 parts by weight or less, or 1 part by weight or less, based on 100 parts by weight of the polyester containing a repeating unit derived from 1,4-butanediol or the polymer blend containing the polyester. If the amount of the tin catalyst charged is too small, thermal decomposition of the polyester or polymer blend may not occur, and if the amount of the tin catalyst charged is too large, the economic efficiency may deteriorate due to the excessive charge.

Moreover, the thermal decomposition is carried out at a temperature of 400°C or more. The thermal decomposition may be carried out at a temperature of 400°C or more, for example, at a temperature of 400°C or more, 420°C or more, 440°C or more, 460°C or more, 480°C or more, or 500°C or more, and 800°C or less, 700°C or less, 650°C or less, 630°C or less, 600°C or less, or 550°C or less. If the thermal decomposition temperature is too low, the thermal decomposition of the polyester or polymer blend may not occur, which may make it difficult to recover butadiene, and if the thermal decomposition temperature is too high, a lot of unexpected impurities may be generated.

Moreover, the thermal decomposition is carried out under a solvent-free condition. For example, when the thermal decomposition is carried out under a solvent-free condition, a polyester or a polymer blend containing a repeating unit derived from 1,4-butanediol may be thermally decomposed without being not dissolved in a solvent. When a solvent is placed in a reactor, impurities may be formed during the process of thermally decomposing a polyester containing a repeating unit derived from 1,4-butanediol or a polymer blend containing the polyester, and a solvent removal process and an additional impurity removal process may be required, which may make the process complicate or require additional equipment. Further, side reactions may occur during the process of removing the solvent, which may lead to a decrease in the recovery rate and purity of monomers. Further, there is a disadvantage that the economic efficiency is lowered due to the use of additional solvents.

Further, the polyester containing a repeating unit derived from 1,4-butanediol is polybutylene adipate terephthalate(PBAT).

The polyester containing a repeating unit derived from 1,4-butanediol is the polybutylene adipate terephthalate, wherein the polybutylene adipate terephthalate may be an aliphatic/aromatic copolyester manufactured using 1,4-butanediol as an aliphatic glycol, adipic acid, which is an aliphatic component as dicarboxylic acid, and dimethyl terephthalate, which is an aromatic component as a raw material.

The polybutylene adipate terephthalate has a weight average molecular weight(Mw) of 50,000 to 300,000 g/mol, which is measured using a gel permeation chromatography(GPC), and more specifically, it may have a weight average molecular weight of 50,000 g/mol or more, 70,000 g/mol or more, or 100,000 g/mol or more, and 300,000 g/mol or less, or 200,000 g/mol or less, or 150,000 g/mol or less. If the weight average molecular weight of the polybutylene adipate terephthalate is too small, the overall mechanical properties may deteriorate significantly, and if the weight average molecular weight is too large, the progress of processes may be difficult, and processability and elongation may be reduced.

The polymer blend may be a polymer blend of polylactic acid and polybutylene adipate terephthalate, or a polymer blend of hydroxyalkanoate copolymer and polybutylene adipate terephthalate.

The hydroxyalkanoate copolymer may include two or more types of repeating units selected from the group consisting of a repeating unit derived from 3-hydroxypropionic acid, a repeating unit derived from lactic acid or lactide, and a repeating unit derived from glycolic acid or glycolide. Further, in order to recover a large amount of butadiene, and the like, the hydroxyalkanoate copolymer may be 3-hydroxypropionate-lactide copolymer, glycolide-lactide copolymer or 3-hydroxypropionate-glycolide copolymer.

Further, the hydroxyalkanoate copolymer may be a block copolymer containing two or more types of blocks selected from the group consisting of a block containing a repeating unit derived from 3-hydroxypropionic acid, a block containing a repeating unit derived from lactic acid or lactide, and a block containing a repeating unit derived from glycolic acid or glycolide. Further, in order to recover butadiene and the like with high purity and high yield, the hydroxyalkanoate copolymer may be 3-hydroxypropionate-lactide block copolymer, glycolide-lactide block copolymer, or 3-hydroxypropionate-glycolide block copolymer.

In the method of manufacturing butadiene according to one embodiment, the polyester containing a repeating unit derived from 1,4-butanediol or a polymer blend containing the polyester may be a polymer blend comprising polylactic acid and a polyester containing a repeating unit derived from 1,4-butanediol.

The step of thermally decomposing a polyester containing a repeating unit derived from 1,4-butanediol or a polymer blend containing the polyester to manufacture butadiene may comprise,
performing a first thermal decomposition of the polymer blend comprising a polylactic acid and a polyester containing a repeating unit derived from 1,4-butanediol to produce lactide; and
performing a second thermal decomposition of the primarily decomposed polymer blend containing a polylactic acid and the polyester containing a repeating unit derived from 1,4-butanediol to manufacture butadiene.

Before the step of performing a first thermal decomposition of the polymer blend comprising a polylactic acid and a polyester containing a repeating unit derived from 1,4-butanediol to produce lactide, the method may further comprise a step of melting the polymer blend comprising polylactic acid and a polyester containing a repeating unit derived from 1,4-butanediol.

The polylactic acid included in the polymer blend may be produced by subjecting lactic acid or lactide to fermentation or polycondensation. The lactide can be divided into L-lactide consisting of L-lactic acid, D-lactide consisting of D-lactic acid, and meso-lactide consisting of one L-form and one D-form. Further, a mixture of L-lactide and D-lactide in a weight ratio of 50:50 may be used as D,L-lactide or rac-lactide. If polymerization proceeds using only L-lactide or D-lactide with high optical purity among these lactides, L- or D-polylactide (PLLA or PDLA) with very high stereoregularity can be obtained, and such a polylactide has a faster crystallization rate and may also have a higher crystallization rate than polylactide with low optical purity.

The polylactic acid has a weight average molecular weight(Mw) of 50,000 to 300,000 g/mol, which is measured using a gel permeation chromatography (GPC), and more specifically, it has a weight average molecular weight of 50,000 g/mol or more, 70,000 g/mol or more, or 100,000 g/mol or less, and 300,000 g/mol or less, or 200,000 g/mol or less, or 150,000 g/mol or less. If the weight average molecular weight of the polylactic acid is too small, the overall mechanical properties may be significantly reduced, and if the weight average molecular weight is too large, the progress of processes may be difficult, and the processability and elongation may be reduced.

On the other hand, the lactic acid or lactide may be a plastic and biodegradable compound produced from recyclable sources by microbial fermentation, and the polylactic acid formed by polymerizing it may also contain a large amount of bio raw materials while exhibiting environmental friendliness and biodegradability.

Further, the lactide produced by thermally decomposing a polymer blend containing polylactic acid containing the bio raw material may also contain a lot of bio raw material.

The polymer blend can be produced by compounding the polylactic acid and the polyester containing a repeating unit derived from 1,4-butanediol in a molten state.

The weight ratio of the polylactic acid contained in the polymer blend and the polyester containing a repeating unit derived from 1,4-butanediol may be 1:99 to 99:1, 5:95 to 95:5, 10:90 to 90:10, 15:85 to 85:15, 20:80 to 80:20, 25:75 to 75:25, or 30 :70 to 70:30.

The first and second thermal decompositions can be carried out under a solvent-free condition, or may be carried out under a tin catalyst. On the other hand, the first thermal decomposition may be carried out at a temperature of 220°C or more and 300°C or less, for example, at a temperature of 220°C or more, 230°C or more, 240°C or more, 250°C or more, and 300°C or less, 290°C or less, and 280°C or less. If the first decomposition temperature is too low, thermal decomposition of the polymer blend may not occur, and if the first thermal decomposition temperature is too high, a lot of unexpected impurities may be generated.

Further, the first thermal decomposition may be carried out under a pressure of more than 0.01 torr and less than 50 torr, for example, under a pressure of more than 0.01 torr, 0.05 torr or more, 0.1 torr or more, 0.5 torr or more, 1 torr or more, 2 torr or more, 4 torr or more, or 5 torr or more, and 50 torr or less, 40 torr or less, 30 torr or less, or 20 torr or less, but is not limited thereto. As the thermal decomposition pressure is lower, the separation and recovery of lactide can become easier.

After the thermally decomposing the polymer blend to produce lactide, the lactide can be separated by distillation under reduced pressure.

For example, the first thermal decomposition may allow a thermal decomposition to occur under a reduced pressure condition which is carried out under a pressure of more than 1 torr, so that the lactide produced at this time can be recovered through distillation under reduced pressure. Further, even if the first thermal decomposition is not carried out under a reduced pressure condition, the lactide can be recovered through distillation.

The polymer blend remaining after the recovery of lactide can be subjected to second thermal decomposition to manufacture butadiene.

The second thermal decomposition may be carried out at a temperature of 400°C or more, for example, at a temperature of 400°C or more, 420°C or more, 440°C or more, 460°C or more, 480°C or more, or 500°C or more, and 800°C or less, 700°C or less, 650°C or less, 630°C or less, 600°C or less, or 550°C or less. If the second thermal decomposition temperature is too low, second thermal decomposition of the polymer blend may not occur, which may make it difficult to recover butadiene, and if the second thermal decomposition temperature is too high, a lot of unexpected impurities may be generated.

The difference between the first thermal decomposition temperature and the second thermal decomposition temperature may be 150°C or more and 350°C or less, and, for example, it may be 150°C or more, 160°C or more, 170°C or more, 180°C or more, 190°C or more, 200°C or more, 210°C or more, and 350°C or less, 340°C or less, 330°C or less, 320°C or less, 310°C or less. If the difference between the first thermal decomposition temperature and the second thermal decomposition temperature is too small, it may become difficult to recover the butadiene, and if the difference between the first thermal decomposition temperature and the second thermal decomposition temperature is too large, a lot of unexpected impurities may be generated. The butadiene produced through the second thermal decomposition can be recovered using a gas collecting device.

In the method of manufacturing butadiene according to one embodiment, the step of thermally decomposing a polyester containing a repeating unit derived from 1,4-butanediol or a polymer blend containing the polyester to manufacture butadiene may comprise the steps of:
performing a first thermal decomposition of the polymer blend comprising a 3-hydroxypropionate-lactide copolymer and a polyester containing a repeating unit derived from 1,4-butanediol to produce lactide;
performing a second thermal decomposition of the primarily decomposed polymer blend comprising a 3-hydroxypropionate-lactide copolymer and a polyester containing a repeating unit derived from 1,4-butanediol to produce acrylic acid; and
performing a third thermal decomposition of the secondarily decomposed polymer blend comprising a 3-hydroxypropionate-lactide copolymer and a polyester containing a repeating unit derived from 1,4-butanediol to manufacture butadiene.

In addition, before the step of performing a first thermal decomposition of the polymer blend comprising a 3-hydroxypropionate-lactide copolymer and a polyester containing a repeating unit derived from 1,4-butanediol to produce lactide, the method may further comprise a step of melting the polymer blend comprising a 3-hydroxypropionate-lactide copolymer and a polyester containing a repeating unit derived from 1,4-butanediol.

The 3-hydroxypropionate-lactide copolymer may be a block copolymer obtained by polymerizing a polylactic acid prepolymer and a poly(3-hydroxypropionate) prepolymer. The 3-hydroxypropionate-lactide copolymer not only exhibits excellent tensile strength and elastic modulus characteristics possessed by the polylactic acid prepolymer, and also lowers the glass transition temperature(Tg) by the poly(3-hydroxypropionate) prepolymer, increases flexibility and improves mechanical properties such as impact strength, which can prevent the brittleness properties that polylactic acid has poor elongation and easily cracks.

The polylactic acid prepolymer may be prepared by subjecting lactic acid to a fermentation or polycondensation. The polylactic acid prepolymer may have a weight average molecular weight of 1,000 g/mol or more, or 5,000 g/mol or more, or 6,000 g/mol or more, or 8,000 g/mol or more, and 50,000 g/mol or less, or 30,000 g/mol or less. If it is desired to increase the crystallinity of blocks containing repeating units derived from a polylactic acid prepolymer in the final prepared block copolymer, the polylactic acid prepolymer preferably has a high weight average molecular weight of more than 20,000 g/mol, or 22,000 g/mol or more, or 23,000 g/mol or more, or 25,000 g/mol or more, and 50,000 g/mol or less, or 30,000 g/mol or less, or 28,000 g/mol or less, or 26,000 g/mol or less.

If the weight average molecular weight of the polylactic acid prepolymer is 20,000 g/mol or less, the crystals of the polymer are small, which makes it difficult to maintain the crystallinity of the polymer in the final prepared block copolymer. If the weight average molecular weight of the polylactic acid prepolymer is more than 50,000 g/mol, the rate of side reactions occurring inside the prepolymer chain becomes faster than the reaction rate between the polylactic acid prepolymers during polymerization. On the other hand, the term 'lactic acid' as used herein refers to L-lactic acid, D-lactic acid, or a mixture thereof.

The poly(3-hydroxypropionate) prepolymer may be prepared by subjecting 3-hydroxypropionate to a fermentation or a polycondensation polymerization. The weight average molecular weight of the poly(3-hydroxypropionate) prepolymer may be 1,000 g/mol or more, or 5,000 g/mol or more, or 8,000 g/mol or more, or 8,500 g/mol or more, and 50,000 g/mol or less, or 30,000 g/mol or less. If it is intended to increase the crystallinity of repeating units derived from poly(3-hydroxypropionate) prepolymer in the final prepared block copolymer, the poly(3-hydroxypropionate) prepolymer preferably have a high weight average molecular weight of more than 20,000 g/mol, or 22,000 g/mol or more, or 25,000 g/mol or more, and 50,000 g/mol or less, or 30,000 g/mol or less, or 28,000 g/mol or less.

As explained for the polylactic acid prepolymer, if the weight average molecular weight of the poly(3-hydroxypropionate) prepolymer is 20,000 g/mol or less, the crystals of the polymer are small, which makes it difficult to maintain the crystallinity of the polymer in the final prepared block copolymer, and if the weight average molecular weight of the poly(3-hydroxypropionate) prepolymer is more than 50,000 g/mol, the rate of side reaction occurring inside the prepolymer chain becomes faster than the reaction rate between poly(3-hydroxypropionate) prepolymers during polymerization.

That is, at least one of the polylactic acid prepolymer and the poly(3-hydroxypropionate) prepolymer may have a weight average molecular weight of more than 20,000 g/mol and 50,000 g/mol or less.

The 3-hydroxypropionate-lactide copolymer is a block copolymer obtained by polymerizing a polylactic acid prepolymer and a poly(3-hydroxypropionate) prepolymer. In the block copolymer, a weight ratio of the polylactic acid prepolymer and the poly(3-hydroxypropionate) prepolymer may be 95:5 to 50:50, 90:10 to 55:45, 90:10 to 60:40, 90: 10 to 70:30 or 90:10 to 80:20. If the poly(3-hydroxypropionate) prepolymer is included in an excessively small amount relative to the polylactic acid prepolymer, brittleness properties may increase, and if the poly(3-hydroxypropionate) prepolymer is included in an excessively large amount relative to the polylactic acid prepolymer, the molecular weight is lowered, and the processability and heat resistance stability may be reduced.

The hydroxyalkanoate-lactide copolymer has a weight average molecular weight(Mw) of 50,000 to 300,000 g/mol, which is measured using a gel permeation chromatography(GPC), and more specifically, it has a weight average molecular weight of 50,000 g/mol or more, 70,000 g/mol or more, or 100,000 g/mol or more, and 300,000 g/mol or less, or 200,000 g/mol or less, or 150,000 g/mol or less. If the weight average molecular weight of the hydroxyalkanoate-lactide copolymer is too small, the overall mechanical properties may decrease significantly, and if the weight average molecular weight is too large, the progress of processes may be difficult and the processability and elongation may be reduced.

On the other hand, the lactic acid and 3-hydroxypropionate may be plastic and biodegradable compounds prepared from recyclable sources by microbial fermentation, and the block copolymer comprising polylactic acid prepolymer and poly(3-hydroxypropionate) prepolymer formed by polymerizing the same may also contain a large amount of bio raw materials while exhibiting environmental friendliness and biodegradability.

In addition, the lactide and acrylic acid produced by thermally decomposing a copolymer containing the bio raw materials may also contain a large amount of bio raw materials.

The polymer blend can be prepared by compounding the 3-hydroxypropionate-lactide copolymer with a polyester containing a repeating unit derived from 1,4-butanediol in a molten state.

The weight ratio of the 3-hydroxypropionate-lactide copolymer and the polyester containing a repeating unit derived from 1,4-butanediol contained in the polymer blend may be 1:99 to 99:1, 5:95 to 95:5, 10:90 to 90:10, 15:85 to 85:15, 20:80 to 80:20, 25:75 to 75:25, or 30 :70 to 70:30.

The first, second and third thermal decomposition may be carried out under a solvent-free condition, or may be carried out under a tin catalyst. On the other hand, the first thermal decomposition may be carried out at a temperature of 200°C or more and 250°C or less, for example, at a temperature of 200°C or more, 210°C or more, 220°C or more, and 250°C or less, 240°C or less, or 230°C or less. If the first thermal decomposition temperature is too low, thermal decomposition of the polymer blend may not occur, and if the first thermal decomposition temperature is too high, a lot of unexpected impurities may be generated.

In addition, the first thermal decomposition may be carried out under a pressure of more than 0.01 torr and less than 50 torr, for example, under a pressure of more than 0.01 torr, 0.05 torr or more, 0.1 torr or more, 0.5 torr or more, 1 torr or more, 2 torr or more, 4 torr or more, or 5 torr or more, and 50 torr or less, 40 torr or less, 30 torr or less, or 20 torr or less, but is not limited thereto. As the thermal decomposition pressure is lower, the separation and recovery of lactide can become easier.

After the step of performing a first thermal decomposition of the polymer blend to produce lactide, the lactide can be separated by distillation under reduced pressure. For example, the first thermal decomposition may allow a thermal decomposition to occur under a reduced pressure condition which is carried out under a pressure of more than 1 torr, so that the lactide produced at this time can be recovered through distillation under reduced pressure. Further, even if the first thermal decomposition is not carried out under a reduced pressure condition, the lactide can be recovered through distillation.

The polymer blend remaining after the recovery of lactide can be subjected to second thermal decomposition to produce acrylic acid.

On the other hand, the second thermal decomposition may be carried out at a temperature of 260°C or more and 350°C or less, for example, at a temperature of 260°C or more, 270°C or more, 280°C or more, 290°C or more, and 350°C or less, 340°C or less, and 330°C or less. If the second thermal decomposition temperature is too low, second thermal decomposition of the polymer blend may not occur, and thus acrylic acid may not be produced. If the second thermal decomposition temperature is too high, a lot of unexpected impurities may be generated.

The polymer blend remaining after the recovery of acrylic acid can be subjected to third thermal decomposition to manufacture butadiene.

The third thermal decomposition may be carried out at a temperature of 400°C or more, for example, at a temperature of 400°C or more, 420°C or more, 440°C or more, 460°C or more, 480°C or more, or 500°C or more, and 800°C or less, 700°C or less, 650°C or less, 630°C or less, 600°C or less, or 550°C or less. If the third thermal decomposition temperature is too low, third thermal decomposition of the polymer blend may not occur, which may make it difficult to recover butadiene, and if the third thermal decomposition temperature is too high, a lot of unexpected impurities may be generated.

After the step of performing a second thermal decomposition of the primarily decomposed polymer blend to produce acrylic acid, the acrylic acid can be separated by distillation under reduced pressure. For example, the second thermal decomposition may allow a thermal decomposition to occur under a reduced pressure condition which is carried out under a pressure of more than 1 torr, so that the acrylic produced at this time can be recovered through distillation under reduced pressure. Further, even if the second thermal decomposition is not carried out under a reduced pressure condition, the acrylic acid can be recovered through distillation.

On the other hand, the difference between the first thermal decomposition temperature and the second thermal decomposition temperature may be 20°C or more 100°C or less, and for example, it may be 20°C or more, 30°C or more, 40°C or more, 50°C or more, and 100°C or less, 90°C or less, 80°C or less, 70°C or less. If the difference between the first thermal decomposition temperature and the second thermal decomposition temperature is too small, it may become difficult to recover the acrylic acid, and if the difference between the first thermal decomposition temperature and the second thermal decomposition temperature is too large, a lot of unexpected impurities may be generated.

The polymer blend remaining after the recovery of acrylic acid can be subjected to third thermal decomposition to manufacture butadiene.

The third thermal decomposition may be carried out at a temperature of 400°C or more, for example, at a temperature of 400°C or more, 420°C or more, 440°C or more, 460°C or more, 480°C or more, or 500°C or more, and 800°C or less, 700°C or less, 650°C or less, 630°C or less, 600°C or less, or 550°C or less. If the third thermal decomposition temperature is too low, third thermal decomposition of the polymer blend may not occur, which may make it difficult to recover butadiene, and if the third thermal decomposition temperature is too high, a lot of unexpected impurities may be generated.

The difference between the second thermal decomposition temperature and the third thermal decomposition temperature may be 150°C or more and 350°C or less, and for example, it may be 150°C or more, 160°C or more, 170°C or more, 180°C or more, 190°C or more, 200°C or more, or 210°C or more, and 350°C or less, 340°C or less, 330°C or less, 320°C or less, or 310°C or less. If the difference between the second thermal decomposition temperature and the third thermal decomposition temperature is too small, it may become difficult to recover the butadiene, and if the difference between the second thermal decomposition temperature and the third thermal decomposition temperature is too large, a lot of unexpected impurities may be generated. The butadiene produced through the third thermal decomposition can be recovered using a gas collecting device.

In the method of manufacturing butadiene according to one embodiment, the polyester containing a repeating unit derived from 1,4-butanediol or a polymer blend containing the polyester may be a glycolide-lactide copolymer and a polyester containing a repeating unit derived from 1,4-butanediol.

Further, the step of thermally decomposing a polyester containing a repeating unit derived from 1,4-butanediol or a polymer blend containing the polyester to manufacture butadiene may comprise the steps of:
performing a first thermal decomposition of the polymer blend comprising a glycolide-lactide copolymer and a polyester containing a repeating unit derived from 1,4-butanediol to produce lactide and glycolide; and
performing a second thermal decomposition of the primarily decomposed polymer blend comprising a glycolide-lactide copolymer and a polyester containing a repeating unit derived from 1,4-butanediol to manufacture butadiene.

In addition, before the step of performing a first thermal decomposition of the polymer blend comprising a glycolide-lactide copolymer and a polyester containing a repeating unit derived from 1,4-butanediol to produce lactide and glycolide, the method may further comprise a step of melting a polymer blend comprising a glycolide-lactide copolymer and a polyester containing a repeating unit derived from 1,4-butanediol.

The glycolide-lactide copolymer contained in the polymer blend is not particularly limited as long as it is a copolymer obtained by polymerizing a glycolide monomer and a lactide monomer, but for example, it may be a random copolymer obtained by polymerizing a glycolide monomer and a lactide monomer.

Moreover, the glycolide-lactide copolymer may be a block copolymer including one or more polyglycolide blocks and one or more polylactide blocks. That is, the glycolide-lactide copolymer may be a block copolymer obtained by polymerizing a polylactide prepolymer and a polyglycolide prepolymer. As the glycolide-lactide copolymer contains the above-mentioned blocks, it can exhibit the environmental friendliness and biodegradability processed by the polyglycolide and polylactide.

The polylactide prepolymer may be prepared by subjecting lactic acid to a fermentation or polycondensation. The polylactide prepolymer may have a weight average molecular weight of 1,000 g/mol or more, or 5,000 g/mol or more, or 6,000 g/mol or more, or 8,000 g/mol or more, and 50,000 g/mol or less, or 30,000 g/mol or less. If it is desired to increase the crystallinity of blocks containing repeating units derived from a polylactide prepolymer in the final prepared block copolymer, the polylactide prepolymer preferably has a high weight average molecular weight of more than 20,000 g/mol, or 22,000 g/mol or more, or 23,000 g/mol or more, or 25,000 g/mol or more, and 50,000 g/mol or less, or 30,000 g/mol or less, or 28,000 g/mol or less, or 26,000 g/mol or less.

If the weight average molecular weight of the polylactide prepolymer is 20,000 g/mol or less, the crystals of the polymer are small, which makes it difficult to maintain the crystallinity of the polymer in the final prepared block copolymer. If the weight average molecular weight of the polylactide prepolymer is more than 50,000 g/mol, the rate of side reactions occurring inside the prepolymer chain becomes faster than the reaction rate between the polylactide prepolymers during polymerization. On the other hand, the term 'lactic acid' as used herein refers to L-lactic acid, D-lactic acid, or a mixture thereof.

The polyglycolide prepolymer may be prepared by subjecting glycolide to a fermentation or a polycondensation polymerization. The weight average molecular weight of the polyglycolide prepolymer may be 1,000 g/mol or more, or 5,000 g/mol or more, or 8,000 g/mol or more, or 8,500 g/mol or more, and 50,000 g/mol or less, or 30,000 g/mol or less. If it is intended to increase the crystallinity of repeating units derived from polyglycolide prepolymer in the final prepared block copolymer, the polyglycolide prepolymer preferably have a high weight average molecular weight of more than 20,000 g/mol, or 22,000 g/mol or more, or 25,000 g/mol or more, and 50,000 g/mol or less, or 30,000 g/mol or less, or 28,000 g/mol or less.

As explained for the polyglycolide prepolymer, if the weight average molecular weight of the polyglycolide prepolymer is 20,000 g/mol or less, the crystals of the polymer are small, which makes it difficult to maintain the crystallinity of the polymer in the final prepared block copolymer, and if the weight average molecular weight of the polyglycolide prepolymer is more than 50,000 g/mol, the rate of side reaction occurring inside the prepolymer chain becomes faster than the reaction rate between polyglycolide prepolymers during polymerization.

That is, at least one of the polylactide prepolymer and the polyglycolide prepolymer may have a weight average molecular weight of more than 20,000 g/mol and 50,000 g/mol or less.

The glycolide-lactide copolyme is a block copolymer obtained by polymerizing a polylactide prepolymer and a polyglycolide prepolymer. In the block copolymer, a weight ratio of the polylactide prepolymer and the polyglycolide prepolymer may be 90:10 to 30:70, 80:20 to 40:60, or 75:25 to 50:50. If the polyglycolide prepolymer is included in an excessively small amount relative to the polylactide prepolymer, brittleness properties may increase, and if the polyglycolide prepolymer is included in an excessively large amount relative to the polylactide prepolymer, the molecular weight is lowered, and the processability and heat resistance stability may be reduced.

The glycolide-lactide copolymer has a weight average molecular weight(Mw) of 20,000 to 300,000 g/mol, which is measured using a gel permeation chromatography(GPC), and more specifically, it has a weight average molecular weight of 20,000 g/mol or more, 30,000 g/mol or more, 40,000 g/mol or more, 50,000 g/mol or more, 70,000 g/mol or more, or 100,000 g/mol or more, and 300,000 g/mol or less, or 200,000 g/mol or less, or 150,000g/mol or less. If the weight average molecular weight of the glycolide-lactide copolymer is too small, the overall mechanical properties may decrease significantly, and if the weight average molecular weight is too large, the progress of processes may be difficult and the processability and elongation may be reduced.

In the method of producing lactide and glycolide according to one embodiment, the glycolide-lactide copolymer may be, for example, P2066 (lactide:glycolide = 65:35), P2191 (lactide:glycolide = 50:50), P1941 (lactide:glycolide = 75:25), and the like from Sigma-Aldrich, or BD01128150 from BLD Pharm.

The polymer blend can be produced by compounding the glycolide-lactide copolymer and the polyester containing a repeating unit derived from 1,4-butanediol in a molten state.

The weight ratio of the glycolide-lactide copolymer and the polyester containing a repeating unit derived from 1,4-butanediol contained in the polymer blend may be 1:99 to 99:1, 5:95 to 95:5, 10:90 to 90:10, 15:85 to 85:15, 20:80 to 80:20, 25:75 to 75:25, or 30 :70 to 70:30.

The first and second thermal decomposition may be carried out under a solvent-free condition, or may be carried out under a tin catalyst. On the other hand, the first thermal decomposition can be carried out at a temperature of 200°C or more and 380°C or less, for example, at a temperature of 200°C or more, 210°C or more, 220°C or more, and 380°C or less, 300°C or less, and 250°C or less. If the first decomposition temperature is too low, thermal decomposition of the polymer blend may not occur, and if the first thermal decomposition temperature is too high, a lot of unexpected impurities may be generated.

Further, the first thermal decomposition may be carried out under a pressure of more than 0.01 torr and less than 50 torr, for example, under a pressure of more than 0.01 torr, 0.05 torr or more, 0.1 torr or more, 0.5 torr or more, 1 torr or more, 2 torr or more, 4 torr or more, or 5 torr or more, and 50 torr or less, 40 torr or less, 30 torr or less, or 20 torr or less, but is not limited thereto. As the thermal decomposition pressure is lower, the separation and recovery of lactide can become easier.

After the step of thermally decomposing the polymer blend to produce lactide and glycolide, the lactide and glycolide can be separated by distillation under reduced pressure.

For example, the first thermal decomposition may allow a thermal decomposition to occur under a reduced pressure condition which is carried out under a pressure of more than 1 torr, so that the lactide and glycolide produced at this time can be recovered through distillation under reduced pressure. Further, even if the first thermal decomposition is not carried out under a reduced pressure condition, the lactide and glycolide can be recovered through distillation.

The polymer blend remaining after the recovery of lactide and glycolide can be subjected to second thermal decomposition to manufacture butadiene.

The second thermal decomposition may be carried out at a temperature of 400°C or more, for example, at a temperature of 400°C or more, 420°C or more, 440°C or more, 460°C or more, 480°C or more, or 500°C or more, and 800°C or less, 700°C or less, 650°C or less, 630°C or less, 600°C or less, or 550°C or less. If the second thermal decomposition temperature is too low, second thermal decomposition of the polymer blend may not occur, which may make it difficult to recover butadiene. If the second thermal decomposition temperature is too high, a lot of unexpected impurities may be generated.

On the other hand, the difference between the first thermal decomposition temperature and the second thermal decomposition temperature may be 100°C or more and 400°C or less, and for example, it may be 100°C or more, 150 °C or more, 180 °C or more, 200 °C or more, 220 °C or more, 250 °C or more, or 280 °C or more, and 400 °C or less, 380 °C or less, 350 °C or less, 330 °C or less, or 300 °C or less. If the difference between the first thermal decomposition temperature and the second thermal decomposition temperature is too small, it may become difficult to recover the butadiene, and if the difference between the first thermal decomposition temperature and the second thermal decomposition temperature is too large, a lot of unexpected impurities may be generated. The butadiene produced through the second thermal decomposition can be recovered using a gas collecting device.

**In** the method of manufacturing butadiene according to one embodiment, the polyester containing a repeating unit derived from 1,4-butanediol or a polymer blend containing the polyester may be a polymer blend comprising a 3-hydroxypropionate-glycolide copolymer and a polyester containing a repeating unit derived from 1,4-butanediol.

Further, the step of thermally decomposing a polyester containing a repeating unit derived from 1,4-butanediol or a polymer blend containing the polyester to manufacture butadiene may comprise the steps of:
performing a first thermal decomposition of the polymer blend comprising a 3-hydroxypropionate-glycolide copolymer and a polyester containing a repeating unit derived from 1,4-butanediol to produce glycolide;
performing a second thermal decomposition of the primarily decomposed polymer blend comprising a 3-hydroxypropionate-glycolide copolymer and a polyester containing a repeating unit derived from 1,4-butanediol obtained to produce acrylic acid; and
performing a third thermal decomposition of the secondarily decomposed polymer blend comprising a 3-hydroxypropionate-glycolide copolymer and a polyester containing a repeating unit derived from 1,4-butanediol to manufacture butadiene.

**In** addition, before the step of performing a first thermal decomposition of the polymer blend comprising a 3-hydroxypropionate-glycolide copolymer and a polyester containing a repeating unit derived from 1,4-butanediol to produce glycolide, the method may further comprise a step of melting the polymer blend comprising a 3-hydroxypropionate-glycolide copolymer and a polyester containing a repeating unit derived from 1,4-butanediol.

The 3-hydroxypropionate-glycolide copolymer includes a block containing a repeating unit derived from 3-hydroxypropionate and a block containing a repeating unit derived from glycolide. Since these blocks are directly bonded, ester-bonded, amide-bonded, urethane-bonded, or carbonate-bonded, it is possible to complement the disadvantage that the biodegradable resin containing only polyglycolide has low elongation properties. Further, these copolymers can have excellent biodegradability while complementing the mechanical properties of each homopolymer.

The 3-hydroxypropionate-glycolide copolymer includes a block containing a repeating unit derived from 3-hydroxypropionate represented by the following Chemical Formula 2, and may include a block containing a repeating unit derived from glycolide represented by the following Chemical Formula 3.

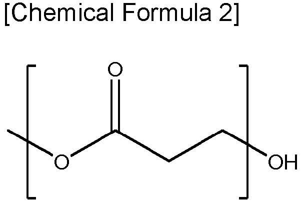

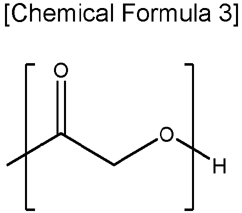

The repeating unit derived from 3-hydroxypropionate represented by Chemical Formula 2 has the advantage that it has excellent mechanical properties and a high glass transition temperature (Tg) of around -20°C, thereby exhibiting a high elongation at break. Therefore, when poly(3-hydroxypropionate) and polyglycolide are chemically bonded to produce a block copolymer, it is possible to produce a biodegradable material with excellent mechanical properties.

In addition, the repeating unit represented by Chemical Formula 2 and the repeating unit represented by Chemical Formula 3 may be connected by a direct bond, an ester bond, an amide bond, a urethane bond, a urea bond, or a carbonate bond. For example, the 3-hydroxypropionate-glycolide copolymer may be a block copolymer represented by the following Chemical Formula 1. 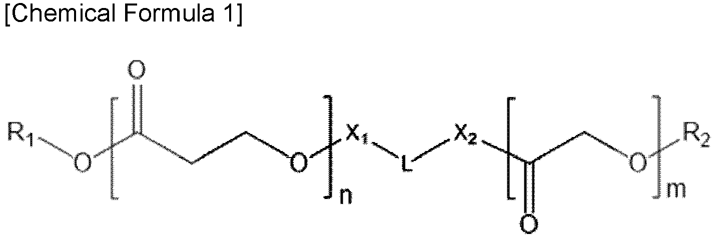 wherein, in Chemical Formula 1,
R₁ and R₂ are each independently hydrogen, N, O, S, or a substituted or unsubstituted C₁₋₂₀ alkyl,
X₁ and X₂ are each independently a direct bond, -COO-, -NR'CO-, -(NR')(COO)-, -R'NCONR'-, or -OCOO-,
R' is each independently hydrogen, or a C₁₋₂₀ alkyl,
L is a direct bond; a substituted or unsubstituted C₁₋₁₀ alkylene; a substituted or unsubstituted C₆₋₆₀ arylene; or a substituted or unsubstituted C₂₋₆₀ heteroarylene containing one or more heteroatoms selected from the group consisting of N, O, and S, and
n and m may each independently be an integer from 1 to 10,000.

The n refers to the number of repetitions of a repeating unit derived from 3-hydroxypropionate, and by introducing it within the above range, it is possible to adjust the physical properties such as elongation while maintaining the inherent physical properties of polyglycolide. Further, the m refers to the number of repetition of the repeating unit derived from glycolide.

Further, the X₁, X₂, and L may be a direct bond.

The Chemical Formula 1 may be represented by the following Chemical Formula 1-1. 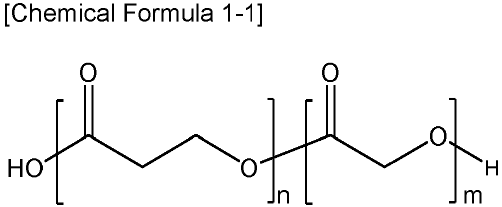 wherein, in Chemical Formula 1-1,
n and m may be as described above.

Preferably, n may be 10 to 700, m may be 10 to 700, n may be 20 or more, 30 or more, 40 or more, 50 or more, or 60 or more, and 650 or less, 600 or less, 550 or less, 500 or less, or 450 or less, and m may be 20 or more, 30 or more, 40 or more, 50 or more, or 60 or more, and 650 or less, 600 or less, 550 or less, 500 or less, or 450 or less.

The 3-hydroxypropionate-glycolide copolymer may have a weight average molecular weight of 10,000 g/mol or more and 500,000 g/mol or less. For example, the weight average molecular weight of the copolymer may be 12,000 g/mol or more, 15,000 g/mol or more, 20,000 g/mol or more, 25,000 g/mol or more, or 30,000 g/mol or more, and 480,000 g/mol or less, 460,000 g/mol or less, 440,000 g/mol or less, or 420,000 g/mol or less.

Further, the 3-hydroxypropionate-glycolide copolymer may be a block copolymer obtained by subjecting a glycolide monomer to ring-opening polymerization in the presence of a poly(3-hydroxypropionate) initiator.

For example, the 3-hydroxypropionate-glycolide copolymer can be prepared through a preparation method comprising the steps of: preparing poly(3-hydroxypropionate) (step 1); and subjecting a glycolide monomer to ring-opening polymerizing in the presence of the poly(3-hydroxypropionate) initiator to prepare a block copolymer (step 2).

Step 1 is a step of preparing the poly(3-hydroxypropionate), wherein the poly(3-hydroxypropionate) means a homopolymer of 3-hydroxypropionic acid, and can be produced by adjusting the degree of polymerization in consideration of the ranges of n and m mentioned above.

The poly(3-hydroxypropionate) initiator may have a weight average molecular weight of 1,000 g/mol or more and 500,000 g/mol or less, 2,000 g/mol or more and 400,000 g/mol or less, 3,000 g/mol or more and 300,000 g/mol or less, 4,000 g/mol or more and 200,000 g/mol or less, 5,000 g/mol or more and 100,000 g/mol or less, 10,000 g/mol or more and 90,000 g/mol or less.

Step 2 may be a step of subjecting a glycolide monomer to ring-opening polymerization using poly(3-hydroxypropionate) as an initiator. Step 2 may be carried out by substantially solvent-free bulk polymerization. At this time, substantially solvent-free can include even the case where a small amount of solvent is used to dissolve the catalyst, for example, the case of using a maximum of less than 1 ml of solvent per kg of monomer used. As the step 2 proceeds by bulk polymerization, it is possible to omit steps such as removing the solvent after polymerization, and it is also possible to suppress decomposition or loss of the resin in this solvent removal step.

The weight ratio of the poly(3-hydroxypropionate) initiator and glycolide monomer may be 1:99 to 99:1, 5:95 to 90:10, 10:90 to 80:20, and 15:85 to 70:30, or 20:80 to 50:50.

On the other hand, since the ring-opening polymerization reaction of glycolide is involved, it can proceed in the presence of a glycolide ring-opening catalyst. For example, the ring-opening catalyst may be a catalyst represented by the following Chemical Formula 4.

[Chemical Formula 4] MA¹ₚA²₂₋ₚ

wherein, in Chemical Formula 4,
M is Al, Mg, Zn, Ca, Sn, Fe, Y, Sm, Lu, Ti or Zr,
p is an integer from 0 to 2,
A¹ and A² may each independently be an alkoxy group or a carboxyl group.

For example, the catalyst represented by Chemical Formula 4 may be tin(II) 2-ethylhexanoate (Sn(Oct)₂).

The preparation of the 3-hydroxypropionate-glycolide copolymer may be carried out at a temperature of 150 to 200°C for 5 minutes to 10 hours or for 10 minutes to 1 hour.

The polymer blend can be prepared by compounding the 3-hydroxypropionate-glycolide copolymer and the polyester containing a repeating unit derived from 1,4-butanediol in a molten state.

The weight ratio of the 3-hydroxypropionate-glycolide copolymer and the polyester containing a repeating unit derived from 1,4-butanediol contained in the polymer blend may be 1:99 to 99:1, 5:95 to 95:5, 10:90 to 90:10, 15:85 to 85:15, 20:80 to 80:20, 25:75 to 75:25, or 30 :70 to 70:30.

The first, second, and third thermal decomposition may be carried out under a solvent-free condition, or may be carried out under a tin catalyst. The first thermal decomposition may be carried out at a temperature of 200°C or more and 250 °C or less, for example, at a temperature of 200°C or more, 210°C or more, 220°C or more, and 250°C or less, 240°C or less, and 230°C or less. If the first thermal temperature is too low, thermal decomposition of the polymer blend may not occur, and if the first thermal decomposition temperature is too high, a lot of unexpected impurities may be generated.

Further, the first thermal decomposition may be carried out under a pressure of more than 0.01 torr and less than 50 torr, for example, under a pressure of more than 0.01 torr, 0.05 torr or more, 0.1 torr or more, 0.5 torr or more, 1 torr or more, 2 torr or more, 4 torr or more, 5 torr or more, and 50 torr or less, 40 torr or less, 30 torr or less, 20 torr or less, but is not limited thereto. As the thermal decomposition pressure is lower, the separation and recovery of glycolide can become easier.

After the step of performing a first thermal decomposition of the polymer blend to produce glycolide, the glycolide can be separated by distillation under reduced pressure.

For example, the first thermal decomposition may allow a thermal decomposition to occur under a reduced pressure condition which is carried out under a pressure of more than 1 torr, so that the glycolide produced at this time can be recovered through distillation under reduced pressure. Further, even if the first thermal decomposition is not carried out under a reduced pressure condition, the glycolide can be recovered through distillation.

The polymer blend remaining after the recovery of glycolide can be subjected to second thermal decomposition to produce acrylic acid.

On the other hand, the second thermal decomposition may be carried out at a temperature of 260°C or more and 380°C or less, for example, at a temperature of 260°C or more, 270°C or more, 280°C or more, 290°C or more, and 380°C or less, 350°C or less, and 330°C or less. If the second thermal decomposition temperature is too low, second thermal decomposition of the polymer blend may not occur, and thus acrylic acid may not be produced. If the second thermal decomposition temperature is too high, a lot of unexpected impurities may be generated.

The polymer blend remaining after the recovery of acrylic acid can be subjected to third thermal decomposition to manufacture butadiene.

The third thermal decomposition may be carried out at a temperature of 400°C or more, for example, at a temperature of 400°C or more, 420°C or more, 440°C or more, 460°C or more, 480°C or more, or 500°C or more, and 800°C or less, 700°C or less, 650°C or less, 630°C or less, 600°C or less, or 550°C or less. If the third thermal decomposition temperature is too low, third thermal decomposition of the polymer blend may not occur, which may make it difficult to recover butadiene, and if the third thermal decomposition temperature is too high, a lot of unexpected impurities may be generated.

After the step of performing a second thermal decomposition of the primarily decomposed polymer blend to produce acrylic acid, the acrylic acid can be separated by distillation under reduced pressure.

For example, the second thermal decomposition may allow a thermal decomposition to occur under a reduced pressure condition which is carried out under a pressure of more than 1 torr, so that the acrylic produced at this time can be recovered through distillation under reduced pressure. Further, even if the second thermal decomposition is not carried out under a reduced pressure condition, the acrylic acid can be recovered through distillation.

On the other hand, the difference between the first thermal decomposition temperature and the second thermal decomposition temperature may be 20°C or more 100°C or less, and for example, it may be 20°C or more, 30°C or more, 40°C or more, 50°C or more, and 100°C or less, 90°C or less, 80°C or less, 70°C or less. If the difference between the first thermal decomposition temperature and the second thermal decomposition temperature is too small, it may become difficult to recover the acrylic acid, and if the difference between the first thermal decomposition temperature and the second thermal decomposition temperature is too large, a lot of unexpected impurities may be generated.

The difference between the second thermal decomposition temperature and the third thermal decomposition temperature may be 150°C or more and 350°C or less, and for example, it may be 150°C or more, 160°C or more, 170°C or more, 180°C or more, 190°C or more, 200°C or more, or 210°C or more, and 350°C or less, 340°C or less, 330°C or less, 320°C or less, or 310°C or less. If the difference between the second thermal decomposition temperature and the third thermal decomposition temperature is too small, it may become difficult to recover the butadiene, and if the difference between the second thermal decomposition temperature and the third thermal decomposition temperature is too large, a lot of unexpected impurities may be generated. The butadiene produced through the third thermal decomposition can be recovered using a gas collecting device.

In the method of manufacturing butadiene according to one embodiment, the polyester containing a repeating unit derived from 1,4-butanediol or a polymer blend containing the polyester may be a polyester containing a repeating unit derived from 1,4-butanediol.

Further, the step of thermally decomposing a polyester containing a repeating unit derived from 1,4-butanediol or a polymer blend containing the polyester to manufacture butadiene may comprise the steps of:
performing a first thermal decomposition of a polyester containing a repeating unit derived from 1,4-butanediol to produce 1,4-butanediol; and
performing a second thermal decomposition of the primarily decomposed polyester containing a repeating unit derived from 1,4-butanediol to manufacture butadiene.

Before the step of performing a first thermal decomposition of the polyester containing a repeating unit derived from 1,4-butanediol to produce 1,4-butanediol, the method may further include a step of melting the polyester containing a repeating unit derived from 1,4-butanediol.

The first and second thermal decomposition can be carried out under a solvent-free condition, or may be carried out under a tin catalyst. The first thermal decomposition may be carried out at a temperature of 220°C or more and 350°C or less, for example, at a temperature of 220°C or more, 230°C or more, 240°C or more, 250°C or more, and 350°C or less, 340°C or less, 330°C or less, 320°C or less, 310°C or less, and 300°C or less. If the first thermal decomposition temperature is too low, thermal decomposition of the polyester containing a repeating unit derived from 1,4-butanediol may not occur, and if the first thermal decomposition temperature is too high, a lot of unexpected impurities may be generated.

Further, the first thermal decomposition may be carried out under a pressure of more than 0.01 torr and less than 50 torr, for example, under a pressure of more than 0.01 torr, 0.05 torr or more, 0.1 torr or more, 0.5 torr or more, 1 torr or more, 2 torr or more, 4 torr or more, or 5 torr or more, and 50 torr or less, 40 torr or less, 30 torr or less, or 20 torr or less. As the thermal decomposition pressure is lower, the separation and recovery of 1,4-butanediol can become easier.

After the step of performing a first thermal decomposition of the polyester containing a repeating unit derived from 1,4-butanediol to produce 1,4-butanediol, the 1,4-butanediol can be separated by distillation or reduced pressure distillation.

For example, the first thermal decomposition may allow a thermal decomposition to occur under a reduced pressure condition which is carried out under a pressure of more than 1 torr, so that the 1,4-butanediol produced at this time can be recovered through distillation under reduced pressure. Further, even if the first thermal decomposition is not carried out under a reduced pressure condition, the 1,4-butanediol can be recovered through distillation.

The 1,4-butanediol is recovered and then the remaining polyester containing a repeating unit derived from 1,4-butanediol can be subjected to second thermal decomposition to manufacture butadiene. The second thermal decomposition may be carried out at a temperature of 400°C or more, for example, at a temperature of 400°C or more, 420°C or more, 440°C or more, 460°C or more, 480°C or more, or 500°C or more, and 800°C or less, 700°C or less, 650°C or less, 630°C or less, 600°C or less, or 550°C or less. If the second thermal decomposition temperature is too low, thermal decomposition of the primarily decomposed polyester containing a repeating unit derived from 1,4-butanediol may not occur, which may make it difficult to recover butadiene, and if the second thermal decomposition temperature is too high, a lot of unexpected impurities may be generated.

The difference between the first thermal decomposition temperature and the second thermal decomposition temperature may be 100°C or more and 300°C or less, and, for example, it may be 100°C or more, 120°C or more, 140°C or more, 160°C or more, 180°C or more, or 200°C or more, and 300°C or less, 280°C or less, 270°C or less, 260°C or less, or 250°C or less. If the difference between the first thermal decomposition temperature and the second thermal decomposition temperature is too small, it may become difficult to recover the butadiene, and if the difference between the first thermal decomposition temperature and the second thermal decomposition temperature is too large, a lot of unexpected impurities may be generated. The butadiene produced through the second thermal decomposition can be recovered using a gas collecting device.

On the other hand, the respective recovery rates of lactide, glycolide, acrylic acid, 1,4-butanediol and/or butadiene may be 30% or more, 50% or more, 60% or more, 70% or more, 80% or more, or 90% or more, for example, 40 to 99.9%, 50 to 99.9%, 60 to 99.9%, 70 to 99.9%, 80 to 99.9%, 90 to 99.9%, 40 to 99%, 50 to 99%, 60 to 99%, 70 to 99%, 80 to 99%, 90 to 99%, 40 to 97%, 50 to 97%, 60 to 97%, 70 to 97%, 80 to 97%, 90 to 97%, 40 to 95%, 50 to 95%, 60 to 95%, 70 to 95%, 80 to 95%, or 90 to 95%. The recovery rate may be calculated on a mole basis.

The respective purities of lactide, glycolide, acrylic acid, 1,4-butanediol and/or butadiene may be 50% or more, 60% or more, 70% or more, 80% or more, or 90% or more, for example, 40 to 99.9%, 50 to 99.9%, 60 to 99.9%, 70 to 99.9%, 80 to 99.9%, 90 to 99.9%, 40 to 99%, 50 to 99%, 60 to 99%, 70 to 99%, 80 to 99%, 90 to 99%, 40 to 97%, 50 to 97%, 60 to 97%, 70 to 97%, 80 to 97%, 90 to 97%, 40 to 95%, 50 to 95%, 60 to 95%, 70 to 95%, 80 to 95%, or 90 to 95%.

### ADVANTAGEOUS EFFECTS

According to the present invention, it is possible to provide a method of manufacturing butadiene, which converts a polyester containing a repeating unit derived from 1,4-butanediol or a polymer blend containing the polyester into recyclable butadiene or the like with high purity and high yield by an environmentally friendly and economical method.

### DETAILED DESCRIPTION OF THE EMBODIMENTS

Hereinafter, the invention will be described in more detail with reference to the following examples. However, these examples are presented for illustrative purposes only, and the scope of the invention is not limited thereby in any way.

### Preparation Example 1: Preparation of polymer blend

70 g of polybutylene adipate terephthalate (PBAT, Solpol 1000N, SOLTECH) and 30 g of polylactic acid (PLA, NatureWorks) were placed in a screw extruder (Thermo scientific, MiniCTW) at 50 g/min, and extruded and mixed at 250°C to prepare a polymer blend.

### Preparation Example 2: Preparation of 3-hydroxypropionate-lactide block copolymer

### (1) Preparation of polylactic acid prepolymer

25 g of 85% L-lactic acid aqueous solution was placed in a 100 ml Schlenk flask in the oil bath, and the pressure was reduced at 70°C and 50 mbar for 2 hours to remove moisture in L-lactic acid. Subsequently, based on 100 parts by weight of lactate, 0.4 parts by weight of p-toluenesulfonic acid (p-TSA) and 0.1 part by weight of SnCl₂ catalyst were added, respectively, and a melt condensation polymerization reaction was carried out at a temperature of 150°C for 12 hours. After the reaction was completed, the reaction product was dissolved in chloroform and then extracted with methanol to obtain a polylactic acid prepolymer (weight average molecular weight: 8,000 g/mol).

### (2) Preparation of poly(3-hydroxypropionate) prepolymer

25 ml of 60% 3-hydroxypropionate aqueous solution was placed in a 100 ml Schlenk flask in an oil bath, moisture in 3-hydroxypropionate was removed at 50°C and 50 mbar for 3 hours. Subsequently, oligomerization was carried out at 70°C and 20 mbar for 2 hours, 0.4 parts by weight of p-toluenesulfonic acid (p-TSA) catalyst based on 100 parts by weight of 3-hydroxypropionate was placed in a reaction flask, and a melt condensation polymerization reaction was carried out at a temperature of 110°C for 24 hours. After the reaction was completed, the reaction product was dissolved in chloroform and then extracted with methanol to obtain poly(3-hydroxypropionate) prepolymer (weight average molecular weight: 26,000 g/mol).

### (3) Preparation of block copolymer

The polylactic acid prepolymer and the poly(3-hydroxypropionate) prepolymer were mixed at a weight ratio of 8:2 in a 100 ml Schlenk flask in an oil bath, and the mixture was added thereto so that the total content was 30 g, and then 90 mg of p-toluenesulfonic acid (p-TSA) was added, and annealed at 60°C for 3 hours. Subsequently, they were mixed using an evaporator at 150°C and 0.5 mbar for 24 hours and subjected to solid phase polymerization reaction to produce a 3-hydroxypropionate-lactide block copolymer (weight average molecular weight: 130,000 g/mol).

On the other hand, the weight average molecular weight of the polylactic acid prepolymer, poly(3-hydroxypropionate) prepolymer, and block copolymer was measured using a gel permeation chromatography (GPC).

### Preparation Example 3: Preparation of polymer blend

70 g of polybutylene adipate terephthalate (PBAT, Solpol 1000N, SOLTECH) and 30 g of 3-hydroxypropionate-lactide block copolymer(PLH) prepared in Preparation Example 2 were placed in a screw extruder (Thermo Scientific, MiniCTW) at 50 g/min, and extruded and mixed at 250°C to prepare a polymer blend.

### Preparation Example 4: Preparation of polymer blend

70 g of polybutylene adipate terephthalate (PBAT, Solpol 1000N, SOLTECH) and 30 g of glycolide-lactide copolymer(PLGA, P1941, Sigma-Aldrich, Lactide: glycolide = 75:25) were placed in a screw extruder(Thermo Scientific, MiniCTW) at 50 g/min, and extruded and mixed at 250°C to prepare a polymer blend.

### Preparation Example 5: Preparation of polymer blend

50 g of polybutylene adipate terephthalate(PBAT, Solpol 1000N, SOLTECH) and 50 g of glycolide-lactide copolymer(PLGA, P1941, Sigma-Aldrich, Lactide: glycolide = 75:25) were placed in a screw extruder(Thermo Scientific, MiniCTW) at 50 g/min, and extruded and mixed at 250°C to prepare a polymer blend.

### Preparation Example 6: Preparation of hydroxyalkanoate-glycolide copolymer

### (1) Preparation of poly(3-hydroxypropionate)

25 ml of 60% 3-hydroxypropionate aqueous solution was placed in a 100 ml Schlenk flask in an oil bath, and moisture in 3-hydroxypropionate was removed at 50°C and 50 mbar for 3 hours. Subsequently, oligomerization was carried out at 70°C and 20 mbar for 2 hours, 0.4 parts by weight of p-toluenesulfonic acid (p-TSA) catalyst based on 100 parts by weight of 3-hydroxypropionate was placed in a reaction flask, and a melt condensation polymerization reaction was carried out at a temperature of 110°C for 24 hours. After the reaction was completed, the reaction product was dissolved in chloroform and extracted with methanol to obtain poly(3-hydroxypropionate) (weight average molecular weight: 26,000 g/mol).

### (2) Preparation of block copolymer

20 g of glycolide, 5 g of poly(3-hydroxypropionate), and 20 mg of tin(II) 2-ethylhexanoate were placed in a 500 mL Teflon-coated round flask, and vacuum-dried at room temperature for 4 hours by applying a vacuum sufficiently. Subsequently, the flask was placed in an oil bath pre-heated to 130°C, the temperature of which was raised to 220°C, and then the ring-opening polymerization reaction was carried out for 30 minutes. After the reaction was completed, the residual monomer was removed through a devolatilization step of the product to obtain a final block copolymer (weight average molecular weight: 130,000 g/mol).

On the other hand, the weight average molecular weight of the poly(3-hydroxypropionate) and the block copolymer was measured using a gel permeation chromatography (GPC).

### Preparation Example 7: Preparation of polymer blend

70 g of polybutylene adipate terephthalate(PBAT, Solpol 1000N, SOLTECH) and 30 g of hydroxyalkanoate-glycolide copolymer(PGH) prepared in Preparation Example 6 were placed in a screw extruder(Thermo Scientific, MiniCTW) at 50 g/min, and extruded and mixed at 250°C to prepare a polymer blend.

### Example 1

10 g of the polymer blend prepared in Preparation Example 1 (the weight ratio of PBAT:PLA is 70:30) was placed in a flask, and dissolved by heating to 250°C. After confirming the dissolution, 0.03 ml of tin 2-ethylhexanoate was added and subjected to a first thermal decomposition reaction, and 2.5 g of lactide was recovered using a distillation device. Subsequently, the temperature inside the reactor was raised to 500°C, the mixture was stirred, and butadiene was collected using a gas collecting device.

### Example 2

10 g of the polymer blend prepared in Preparation Example 1 (the weight ratio of PBAT:PLA is 30:70) was placed in a flask, and dissolved by heating to 250°C. After confirming the dissolution, 0.03 ml of tin 2-ethylhexanoate was added and subjected to a first thermal decomposition reaction. 5.3 g of lactide was recovered using a distillation device. Subsequently, the temperature inside the reactor was raised to 500°C and the mixture was stirred, and butadiene was collected using a gas collecting device.

### Example 3

10 g of the polymer blend prepared in Preparation Example 3 (the weight ratio of PBAT:PLA is 70:30) was placed in a flask, and dissolved by heating to 180°C. After confirming the dissolution, 0.03 ml of tin 2-ethylhexanoate was added thereto. Subsequently, the reaction temperature was raised to 220°C, and then the pressure of the reactor was reduced to 5 torr and subjected to a first thermal decomposition reaction. 4.9 g of lactide was recovered using a distillation device. Subsequently, the temperature inside the reactor was raised to 290°C and the mixture was stirred, and subjected to a second thermal decomposition reaction. 0.8 g of acrylic acid was recovered using a distillation device. Subsequently, the temperature inside the reactor was raised to 500°C and the mixture was stirred, and 0.38 g of butadiene was collected using a gas collecting device.

### Example 4

10 g of the polymer blend prepared in Preparation Example 3 (the weight ratio of PBAT:PLA is 30:70) was placed in a flask, and dissolved by heating to 180°C. After confirming the dissolution, 0.03 ml of tin 2-ethylhexanoate was added thereto. Subsequently, the reaction temperature was raised to 220°C, and then the pressure of the reactor was reduced to 5 torr and subjected to a first thermal decomposition reaction. 2.1 g of lactide was recovered using a distillation device. Subsequently, the temperature inside the reactor was raised to 290°C and the mixture was stirred, and subjected to a second thermal decomposition reaction. 0.4 g of acrylic acid was recovered using a distillation device. Subsequently, the temperature inside the reactor was raised to 500°C and the mixture was stirred, and 0.9 g of butadiene was collected using a gas collecting device.

### Example 5

10 g of the polymer blend prepared in Preparation Example 4 (the weight ratio of PBAT:PLA is 70:30) was placed in a flask, and dissolved by heating to 180°C. After confirming the dissolution, 0.03 ml of tin 2-ethylhexanoate was added thereto. Subsequently, the reaction temperature was raised to 220°C, and then the pressure of the reactor was reduced to 5 torr and subjected to a first thermal decomposition reaction. 2.5 g of lactide and glycolide (recovery rate: 83%) was recovered using a distillation device. Subsequently, the temperature inside the reactor was raised to 500°C and the mixture was stirred, and subjected to a second thermal decomposition reaction. 4.3 g of butadiene (recovery rate: 62%) was recovered using a gas collecting device.

### Example 6

10 g of the polymer blend prepared in Preparation Example 5 (the weight ratio of PBAT:PLA is 50:50) was placed in a flask, and dissolved by heating to 180°C. After confirming the dissolution, 0.03 ml of tin 2-ethylhexanoate was added thereto. Subsequently, the reaction temperature was raised to 220°C, and then the pressure of the reactor was reduced to 5 torr and subjected to a first thermal decomposition reaction. 4.1 g of lactide and glycolide (recovery rate: 81%) was recovered using a distillation device. Subsequently, the temperature inside the reactor was raised to 500°C and the mixture was stirred, and 2.9 g of butadiene (recovery rate: 58%) was recovered using a gas collecting device.

### Example 7

10 g of the polymer blend prepared in Preparation Example 7 (the weight ratio of PBAT:PLA is 70:30) was placed in a flask, and dissolved by heating to 180°C. After confirming the dissolution, 0.03 ml of tin 2-ethylhexanoate was added thereto. Subsequently, the reaction temperature was raised to 220°C, and then the pressure of the reactor was reduced to 5 torr and subjected to a first thermal decomposition reaction, and glycolide was recovered using a distillation device (recovery rate of 81%, purity of 98% or more). Subsequently, the temperature inside the reactor was raised to 290°C and the mixture was stirred, and subjected to a second thermal decomposition reaction, and acrylic acid was recovered using a distillation device (recovery rate of 59%, purity of 99%). Subsequently, the temperature inside the reactor was raised to 500°C and the mixture was stirred, and butadiene was recovered using a gas collecting device (recovery rate of 63%, purity of 99% or more).

### Example 8

10 g of polybutylene adipate terephthalate (PBAT, Solpol 1000N, SOLTECH) was placed in a flask, and dissolved by heating to 180 to 200°C. After confirming the dissolution, 0.03 ml of tin 2-ethylhexanoate was added thereto. Subsequently, the reaction temperature was raised to 250 to 300°C and the mixture was stirred, and subjected to a first thermal decomposition reaction. 1.8 g of 1,4-butanediol was recovered using a distillation device. Subsequently, the temperature inside the reactor was raised to 500°C and the mixture was stirred, and 0.7 g of butadiene was collected using a gas collecting device.

### Example 9

10 g of polybutylene adipate terephthalate(PBAT, Solpol 1000N, SOLTECH) was placed in a flask, and dissolved by heating to 180 to 200°C. After confirming the dissolution, 0.03 ml of tin 2-ethylhexanoate was added thereto. Subsequently, the temperature inside the reactor was raised to 500°C and the mixture was stirred, and 1.1 g of butadiene was collected using a gas collecting device.

### Comparative Example 1

Polylactic acid was placed in a screw extruder(Thermo Scientific, MiniCTW) at 50 g/min, and tin 2-octylate was added at 5 g/min, the internal temperature was raised to 220°C, and distilled under reduced pressure to 15 mmHg to recover lactide.

### Comparative Example 2

2.0 g of poly(propiolactone) and 8.6 mg of hydroquinone monoethyl ether(MEHQ) were mixed, and the was heated under reduced pressure at 210°C and 500 mtorr to obtain 1.39 g of 99.4% acrylic acid.

### Comparative Example 3

10 g of polybutylene adipate terephthalate(PBAT, Solpol 1000N, SOLTECH) was placed in a flask, and dissolved by heating to 180 to 200°C. After confirming the dissolution, 0.03 ml of tin 2-ethylhexanoate was added thereto. Subsequently, the temperature inside the reactor was raised to 280°C and the mixture was stirred, and 1.8 g of 1,4-butanediol was collected using a gas collecting device.

### Evaluation

### 1. Evaluation of recovery rate of monomer

In Examples and Comparative Examples, the recovery rate of lactide, glycolide, 1,4-butanediol, acrylic acid and/or butadiene ((actual recovery amount / theoretical recovery amount) * 100) was calculated, and the results are shown in Tables 1 and 2 below.

### 2. Evaluation of purity of monomer

In Examples and Comparative Examples, the purity of lactide, glycolide, 1,4-butanediol, acrylic acid and/or butadiene was measured using ¹H NMR (400 MHz, CDCl₃), respectively, and the results are shown in Tables 1 and 2 below.
- Lactide (δ, CDCl₃) 1.71 (d, 3H), 5.05 (m, 1H)
- Acrylic acid (δ, CDCl₃) 5.97 (d, 1H), 6.14 (q, 1H), 6.53 (d, 1H)
- Glycolide (δ, CDCl₃) 4.99(s, 4H)
- 1,4-butanediol (δ, CDCl₃) 1.86 (m, 2H), 3.74 (t, 2H)
- Butadiene (δ, CDCl₃) 5.12 (d, 2H), 5.22 (d, 2H), 6.32-6.37 (m, 2H)

**[Table 1]**

| | | Exam | Exam | Exam | Exam | Exam | Exam | Exam | Compara | Compara |
|---|---|---|---|---|---|---|---|---|---|---|
| | | ple 1 | ple 2 | ple 3 | ple 4 | ple 5 | ple 6 | ple 7 | tive Example 1 | tive Example 2 |
| Recov ery rate (%) | Lactide | 83.3 | 75.7 | 87.5 | 87.5 | 83 | 81 | - | 87.0 | - |
| | Glycoli de | - | - | - | - | | | 81 | - | - |
| | Acrylic acid | - | - | 57.1 | 66.7 | - | - | 59 | - | 70.0 |
| | Butadie ne | 68.1 | 46.4 | 52.8 | 53.3 | 62 | 58 | 63 | - | - |
| Purity (%) | Lactide | ≥ 98.0 | ≥ 98.0 | ≥ 98.0 | ≥ 98.0 | > 98 | > 98 | - | ≥ 98.0 | - |
| | Glycoli de | - | - | - | - | > 98 | > 98 | ≥ 98 | - | - |
| | Acrylic acid | - | - | ≥ 98.0 | ≥ 98.0 | - | - | 99 | - | 99.4 |
| | Butadie ne | ≥ 99.0 | ≥ 99.0 | ≥ 98.0 | ≥ 98.0 | > 98 | > 98 | ≥ 99 | - | - |

| [Table 2] | | |
|---|---|---|
| | Example 8 | Example 9 |
| Recovery rate of 1,4-butanediol and butadiene (g) (weight ratio of 1,4-butanediol: butadiene) | 1.8 : 0.7 | 0 : 1.1 |
| Recovery rate of 1,4-butanediol (%) | 44.8 | - |
| Purity of 1,4-butanediol (%) | ≥ 98.0 | - |
| Recovery rate of butadiene (%) | 52.6 | 45.8 |
| Purity of butadiene (%) | ≥ 98.0 | ≥ 98.0 |

According to Tables 1 and 2, it was confirmed that Examples 1 and 2 could recover lactide and butadiene with high purity and high yield, respectively; Examples 3 and 4 could recover lactide, acrylic acid, and butadiene with high purity and high yield, respectively; Examples 5 and 6 could recover lactide and glycolide with a recovery rate of 81% or more, and could recover butadiene with a recovery rate of 58% or more, the purity of each of which exceeded 98%; Example 7 could recover acrylic acid, glycolide , and butadiene with high purity and high yield, respectively; and Examples 8 and 9 could recover 1,4-butanediol and/or butadiene with high purity and high yield, respectively. For reference, after confirming that in the case of Example 8, when PBAT was thermally decomposed at 250 to 300°C, and a lot of PBAT still remained in the residue, the temperature was further raised to 500°C and thermal decomposition was carried out to recover butadiene. On the other hand, it was confirmed that Comparative Example 1 recovered only lactide; Comparative Example 2 recovered only acrylic acid; and Comparative Example 3 recovered only 1,4-butanediol.

## Claims

1. A method of manufacturing butadiene comprising thermally decomposing a polyester containing a repeating unit derived from 1,4-butanediol, or a polymer blend containing the polyester to manufacture butadiene, wherein
the thermal decomposition is carried out under a tin catalyst,
the thermal decomposition is carried out at a temperature of at least 400°C,
the thermal decomposition is carried out under a solvent-free condition, and wherein the tin catalyst is at least one selected from the group consisting of tin(II) 2-ethylhexanoate, tin(II) 2-methylhexanoate, tin(II) 2-propylhexanoate, dioctyltin dilaurate, dihexyltin dilaurate, dibutyltin dilaurate, dipropyltin dilaurate, diethyltin dilaurate, dimetyltin dilaurate, dibutyltin bis(lauryl mercaptide), dimethyltin bis(lauryl mercaptide), diethyltin bis(lauryl mercaptide), dipropyltin bis(lauryl mercaptide), dihexyltin bis(lauryl mercaptide), dioctyltin bis(lauryl mercaptide), dimethyltin bis(isooctylmaleate), diethyltin bis(isooctylmaleate), dipropyltin bis(isooctylmaleate), dibutyltin bis(isooctylmaleate), dihexyltin bis(isooctylmaleate) and dioctyltin bis(isooctylmaleate),
the polyester containing a repeating unit derived from 1,4-butanediol is polybutylene adipate terephthalate(PBAT).

2. The method of manufacturing butadiene according to claim 1,
before the thermally decomposing a polyester containing a repeating unit derived from 1,4-butanediol or a polymer blend containing the same to manufacture butadiene,
further comprising melting the polyester or the polymer blend,
the melting is carried out under a solvent-free condition,
the melting is carried out at a temperature of 150°C or more and 280°C or less.

3. The method of manufacturing butadiene according to claim 1, wherein
the polymer blend is a polymer blend of polylactic acid and polybutylene adipate terephthalate, or a polymer blend of hydroxyalkanoate copolymer and polybutylene adipate terephthalate,
the hydroxyalkanoate copolymer is 3-hydroxypropionate-lactide copolymer, glycolide-lactide copolymer, or 3-hydroxypropionate-glycolide copolymer.

4. The method of manufacturing butadiene according to claim 1, wherein
the thermally decomposing a polyester containing a repeating unit derived from 1,4-butanediol or a polymer blend containing the polyester to manufacture butadiene comprises the steps of,
performing a first thermal decomposition of a polymer blend comprising polylactic acid and the polyester to produce lactide; and
performing a second thermal decomposition of the first decomposed polymer blend to manufacture butadiene.

5. The method of manufacturing butadiene according to claim 4, wherein
the first thermal decomposition is carried out at a temperature of 220°C or more and 300°C or less, and
the second thermal decomposition is carried out at a temperature of at least 400°C or more,
a difference between the first thermal decomposition temperature and the second thermal decomposition temperature is 150°C or more and 350°C or less.

6. The method of manufacturing butadiene according to claim 1, wherein
the thermally decomposing a polyester containing a repeating unit derived from 1,4-butanediol or a polymer blend containing the polyester to manufacture butadiene comprises the steps of:
performing a first thermal decomposition of the polymer blend comprising a 3-hydroxypropionate-lactide copolymer and the polyester to produce lactide;
performing a second thermal decomposition of the first decomposed polymer blend to produce acrylic acid; and
performing a third thermal decomposition of the second decomposed polymer blend to manufacture butadiene.

7. The method of manufacturing butadiene according to claim 6, wherein
the first thermal decomposition is carried out at a temperature of 200°C or more and 250°C or less,
the second thermal decomposition is carried out at a temperature of 260°C or more and 350°C or less, and
the third thermal decomposition is carried out at a temperature of at least 400°C,
a difference between the first thermal decomposition temperature and the second thermal decomposition temperature is 20°C or more and 100°C or less, and
a difference between the second thermal decomposition temperature and the third thermal decomposition temperature is 150°C or more and 350°C or less.

8. The method of manufacturing butadiene according to claim 1, wherein
the thermally decomposing a polyester containing a repeating unit derived from 1,4-butanediol or a polymer blend containing the polyester to manufacture butadiene comprises the steps of:
performing a first thermal decomposition of the polymer blend comprising a glycolide-lactide copolymer and the polyester to produce lactide and glycolide; and
performing a second thermal decomposition of the first decomposed polymer blend to manufacture butadiene.

9. The method of manufacturing butadiene according to claim 8, wherein
the first thermal decomposition is carried out at a temperature of 200°C or more and 380°C or less, and
the second thermal decomposition is carried out at a temperature of at least 400°C,
a difference between the first thermal decomposition temperature and the second thermal decomposition temperature is 100°C or more and 400°C or less.

10. The method of manufacturing butadiene according to claim 1, wherein
the thermally decomposing a polyester containing a repeating unit derived from 1,4-butanediol or a polymer blend containing the polyester to manufacture butadiene comprises the steps of:
performing a first thermal decomposition of the polymer blend comprising a 3-hydroxypropionate-glycolide copolymer and the polyester to produce glycolide;
performing a second thermal decomposition of the first decomposed polymer blend to produce acrylic acid; and
performing a third thermal decomposition of the second decomposed polymer blend to manufacture butadiene.

11. The method of manufacturing butadiene according to claim 10, wherein
the first thermal decomposition is carried out at a temperature of 200°C or more and 250°C or less,
the second thermal decomposition is carried out at a temperature of 260°C or more and 380°C or less, and
the third thermal decomposition is carried out at a temperature of at least 400°C.

12. The method of manufacturing butadiene according to claim 1, wherein
the thermally decomposing a polyester containing a repeating unit derived from 1,4-butanediol or a polymer blend containing the polyester to manufacture butadiene comprises the steps of:
performing a first thermal decomposition of the polyester to produce 1,4-butanediol; and
performing a second thermal decomposition of the first decomposed polyester to manufacture butadiene.

13. The method of manufacturing butadiene according to claim 12, wherein:
the first thermal decomposition is carried out at a temperature of 220°C or more and 350°C or less, and
the second thermal decomposition is carried out at a temperature of at least 400°C,
a difference between the first thermal decomposition temperature and the second thermal decomposition temperature is 100°C or more and 300°C or less.

## Patentansprüche

1. Verfahren zur Herstellung von Butadien, umfassend das thermische Zersetzen eines Polyesters, der eine Wiederholungseinheit enthält, die von 1,4-Butandiol abgeleitet ist, oder einer Polymermischung, die den Polyester enthält, um Butadien herzustellen, wobei
die thermische Zersetzung unter einem Zinnkatalysator durchgeführt wird,
die thermische Zersetzung bei einer Temperatur von mindestens 400 °C durchgeführt wird,
die thermische Zersetzung unter einem lösungsmittelfreien Zustand durchgeführt wird und wobei der Zinnkatalysator mindestens einer ist, der aus der Gruppe ausgewählt ist, die aus Zinn(II)-2-ethylhexanoat, Zinn(II)-2-methylhexanoat, Zinn(II)-2-propylhexanoat, Dioctylzinndilaurat, Dihexylzinndilaurat, Dibutylzinndilaurat, Dipropylzinndilaurat, Diethylzinndilaurat, Dimetylzinndilaurat, Dibutylzinnbis(laurylmercaptid), Dimethylzinnbis(laurylmercaptid), Diethylzinnbis(laurylmercaptid), Dipropylzinnbis(laurylmercaptid), Dihexylzinnbis(laurylmercaptid), Dioctylzinnbis(laurylmercaptid), Dimethylzinnbis(isooctylmaleat), Diethylzinnbis(isooctylmaleat), Dipropylzinnbis(isooctylmaleat), Dibutylzinnbis(isooctylmaleat), Dihexylzinnbis(isooctylmaleat) und Dioctylzinnbis(isooctylmaleat) besteht,
der Polyester, der eine Wiederholungseinheit enthält, die von 1,4-Butandiol abgeleitet ist, Polybutylenadipatterephthalat (PBAT) ist.

2. Verfahren zur Herstellung von Butadien nach Anspruch 1,
vor dem thermischen Zersetzen eines Polyesters, der eine Wiederholungseinheit enthält, die von 1,4-Butandiol abgeleitet ist, oder einer Polymermischung, die dieselbe enthält, um Butadien herzustellen,
ferner umfassend das Schmelzen des Polyesters oder der Polymermischung,
das Schmelzen unter einem lösungsmittelfreien Zustand durchgeführt wird,
das Schmelzen bei einer Temperatur von 150 °C oder mehr und 280 °C oder weniger durchgeführt wird.

3. Verfahren zur Herstellung von Butadien nach Anspruch 1, wobei
die Polymermischung eine Polymermischung aus Polymilchsäure und Polybutylenadipatterephthalat oder eine Polymermischung aus Hydroxyalkanoat-Copolymer und Polybutylenadipatterephthalat ist,
das Hydroxyalkanoat-Copolymer 3-Hydroxypropionat-Lactid-Copolymer, Glycolid-Lactid-Copolymer oder 3-Hydroxypropionat-Glycolid-Copolymer ist.

4. Verfahren zur Herstellung von Butadien nach Anspruch 1, wobei
das thermische Zersetzen eines Polyesters, der eine Wiederholungseinheit enthält, die von 1,4-Butandiol abgeleitet ist, oder einer Polymermischung, die den Polyester enthält, um Butadien herzustellen, die Schritte umfasst,
Durchführen einer ersten thermischen Zersetzung einer Polymermischung, die Polymilchsäure und den Polyester umfasst, um Lactid herzustellen; und
Durchführen einer zweiten thermischen Zersetzung der ersten zersetzten Polymermischung, um Butadien herzustellen.

5. Verfahren zur Herstellung von Butadien nach Anspruch 4, wobei
die erste thermische Zersetzung bei einer Temperatur von 220 °C oder mehr und 300 °C oder weniger durchgeführt wird, und
die zweite thermische Zersetzung bei einer Temperatur von mindestens 400 °C oder mehr durchgeführt wird,
eine Differenz zwischen der ersten thermischen Zersetzungstemperatur und der zweiten thermischen Zersetzungstemperatur 150 °C oder mehr und 350 °C oder weniger beträgt.

6. Verfahren zur Herstellung von Butadien nach Anspruch 1, wobei
das thermische Zersetzen eines Polyesters, der eine Wiederholungseinheit enthält, die von 1,4-Butandiol abgeleitet ist, oder einer Polymermischung, die den Polyester enthält, um Butadien herzustellen, die Schritte umfasst:
Durchführen einer ersten thermischen Zersetzung der Polymermischung, die ein 3-Hydroxypropionat-Lactid-Copolymer und den Polyester umfasst, um Lactid herzustellen;
Durchführen einer zweiten thermischen Zersetzung der ersten zersetzten Polymermischung, um Acrylsäure herzustellen; und
Durchführen einer dritten thermischen Zersetzung der zweiten zersetzten Polymermischung, um Butadien herzustellen.

7. Verfahren zur Herstellung von Butadien nach Anspruch 6, wobei
die erste thermische Zersetzung bei einer Temperatur von 200 °C oder mehr und 250 °C oder weniger durchgeführt wird,
die zweite thermische Zersetzung bei einer Temperatur von 260 °C oder mehr und 350 °C oder weniger durchgeführt wird, und
die dritte thermische Zersetzung bei einer Temperatur von mindestens 400 °C durchgeführt wird,
eine Differenz zwischen der ersten thermischen Zersetzungstemperatur und der zweiten thermischen Zersetzungstemperatur 20 °C oder mehr und 100 °C oder weniger beträgt, und
eine Differenz zwischen der zweiten thermischen Zersetzungstemperatur und der dritten thermischen Zersetzungstemperatur 150 °C oder mehr und 350 °C oder weniger beträgt.

8. Verfahren zur Herstellung von Butadien nach Anspruch 1, wobei
das thermische Zersetzen eines Polyesters, der eine Wiederholungseinheit enthält, die von 1,4-Butandiol abgeleitet ist, oder einer Polymermischung, die den Polyester enthält, um Butadien herzustellen, die Schritte umfasst:
Durchführen einer ersten thermischen Zersetzung der Polymermischung, die ein Glycolid-Lactid-Copolymer und den Polyester umfasst, um Lactid und Glycolid herzustellen; und
Durchführen einer zweiten thermischen Zersetzung der ersten zersetzten Polymermischung, um Butadien herzustellen.

9. Verfahren zur Herstellung von Butadien nach Anspruch 8, wobei
die erste thermische Zersetzung bei einer Temperatur von 200 °C oder mehr und 380 °C oder weniger durchgeführt wird, und
die zweite thermische Zersetzung bei einer Temperatur von mindestens 400 °C durchgeführt wird,
eine Differenz zwischen der ersten thermischen Zersetzungstemperatur und der zweiten thermischen Zersetzungstemperatur 100 °C oder mehr und 400 °C oder weniger beträgt.

10. Verfahren zur Herstellung von Butadien nach Anspruch 1, wobei
das thermische Zersetzen eines Polyesters, der eine Wiederholungseinheit enthält, die von 1,4-Butandiol abgeleitet ist, oder einer Polymermischung, die den Polyester enthält, um Butadien herzustellen, die Schritte umfasst:
Durchführen einer ersten thermischen Zersetzung der Polymermischung, die ein 3-Hydroxypropionat-Glycolid-Copolymer und den Polyester umfasst, um Glycolid herzustellen;
Durchführen einer zweiten thermischen Zersetzung der ersten zersetzten Polymermischung, um Acrylsäure herzustellen; und
Durchführen einer dritten thermischen Zersetzung der zweiten zersetzten Polymermischung, um Butadien herzustellen.

11. Verfahren zur Herstellung von Butadien nach Anspruch 10, wobei
die erste thermische Zersetzung bei einer Temperatur von 200 °C oder mehr und 250 °C oder weniger durchgeführt wird,
die zweite thermische Zersetzung bei einer Temperatur von 260 °C oder mehr und 380 °C oder weniger durchgeführt wird, und
die dritte thermische Zersetzung bei einer Temperatur von mindestens 400 °C durchgeführt wird.

12. Verfahren zur Herstellung von Butadien nach Anspruch 1, wobei
das thermische Zersetzen eines Polyesters, der eine Wiederholungseinheit enthält, die von 1,4-Butandiol abgeleitet ist, oder einer Polymermischung, die den Polyester enthält, um Butadien herzustellen, die Schritte umfasst:
Durchführen einer ersten thermischen Zersetzung des Polyesters, um 1,4-Butandiol herzustellen; und
Durchführen einer zweiten thermischen Zersetzung des ersten zersetzten Polyesters, um Butadien herzustellen.

13. Verfahren zur Herstellung von Butadien nach Anspruch 12, wobei:
die erste thermische Zersetzung bei einer Temperatur von 220 °C oder mehr und 350 °C oder weniger durchgeführt wird, und
die zweite thermische Zersetzung bei einer Temperatur von mindestens 400 °C durchgeführt wird,
eine Differenz zwischen der ersten thermischen Zersetzungstemperatur und der zweiten thermischen Zersetzungstemperatur 100 °C oder mehr und 300 °C oder weniger beträgt.

## Revendications

1. Procédé de fabrication de butadiène consistant à décomposer thermiquement un polyester contenant une unité de répétition dérivée de 1,4-butanediol, ou un mélange de polymères contenant le polyester pour fabriquer du butadiène, dans lequel
la décomposition thermique est effectuée avec un catalyseur à l'étain,
la décomposition thermique est effectuée à une température d'au moins 400°C,
la décomposition thermique est effectuée dans des conditions sans solvant, et dans lequel le catalyseur à l'étain est un ou plusieurs éléments sélectionnés dans le groupe constitué de 2-éthylhexanoate d'étain(II), 2-méthylhexanoate d'étain(II), 2-propylhexanoate d'étain(II), dilaurate de dioctylétain, dilaurate de dihexylétain, dilaurate de dibutylétain, dilaurate de dipropylétain, dilaurate de diéthylétain, dilaurate de diméthylétain, bis(lauryl mercaptide) de dibutylétain, bis(lauryl mercaptide) de diméthylétain, bis(lauryl mercaptide) de dibutylétain, bis(lauryl mercaptide) de dipropylétain, bis(lauryl mercaptide) de dihexylétain, bis(lauryl mercaptide) de dioctylétain, bis(isooctylmaléate) de diméthylétain, bis(isooctylmaléate) de diéthylétain, bis(isooctylmaléate) de dipropylétain, bis(isooctylmaléate) de diéthylétain, bis(isooctylmaléate) de dihexylétain et bis(isooctylmaléate) de dioctylétain,
le polyester contenant une unité de répétition dérivée de 1,4-butanediol est le polybutylène adipate-téréphtalate (PBAT).

2. Procédé de fabrication de butadiène selon la revendication 1,
avant la décomposition thermique d'un polyester contenant une unité de répétition dérivée de 1,4-butanediol ou d'un mélange de polymères contenant celui-ci pour fabriquer du butadiène,
consistant en outre à faire fondre le polyester ou le mélange de polymères,
la fusion est effectuée dans des conditions sans solvant,
la fusion est effectuée à une température égale ou supérieure à 150°C et égale ou inférieure à 280°C.

3. Procédé de fabrication de butadiène selon la revendication 1, dans lequel
le mélange de polymères est un mélange de polymères d'acide polylactique et de polybutylène adipate-téréphtalate, ou un mélange de polymères de copolymère d'hydroxyalcanoate et de polybutylène adipate-téréphtalate,
le copolymère d'hydroxyalcanoate est un copolymère de lactide-3-hydroxypropionate, un copolymère de lactide-glycolide ou un copolymère de glycolide-3-hydroxypropionate.

4. Procédé de fabrication de butadiène selon la revendication 1, dans lequel
la décomposition thermique d'un polyester contenant une unité de répétition dérivée de 1,4-butanediol ou d'un mélange de polymères contenant le polyester pour fabriquer du butadiène comprend les étapes consistant à,
effectuer une première décomposition thermique d'un mélange de polymères comprenant l'acide polylactique et le polyester pour produire du lactide ; et
effectuer une deuxième décomposition thermique du premier mélange de polymères décomposés pour fabriquer du butadiène.

5. Procédé de fabrication de butadiène selon la revendication 4, dans lequel
la première décomposition thermique est effectuée à une température égale ou supérieure à 220°C et égale ou inférieure à 300°C, et
la deuxième décomposition thermique est effectuée à une température d'au moins 400°C ou plus,
une différence entre la première température de décomposition thermique et la deuxième température de décomposition thermique est égale ou supérieure à 150°C et égale ou inférieure à 350°C.

6. Procédé de fabrication de butadiène selon la revendication 1, dans lequel la décomposition thermique d'un polyester contenant une unité de répétition dérivée de 1,4-butanediol ou d'un mélange de polymères contenant le polyester pour fabriquer du butadiène comprend les étapes consistant à :
effectuer une première décomposition thermique du mélange de polymères comprenant un copolymère de lactide-3-hydroxypropionate et le polyester pour produire lactide ;
effectuer une deuxième décomposition thermique du premier mélange de polymères décomposés pour produire de l'acide acrylique ; et
effectuer une troisième décomposition thermique du deuxième mélange de polymères décomposés pour fabriquer du butadiène.

7. Procédé de fabrication de butadiène selon la revendication 6, dans lequel
la première décomposition thermique est effectuée à une température égale ou supérieure à 200°C et égale ou inférieure à 250°C,
la deuxième décomposition thermique est effectuée à une température égale ou supérieure à 260°C et égale ou inférieure à 350°C, et
la troisième décomposition thermique est effectuée à une température d'au moins 400°C,
une différence entre la première température de décomposition thermique et la deuxième température de décomposition thermique est égale ou supérieure à 20°C et égale ou inférieure à 100°C, et
une différence entre la deuxième température de décomposition thermique et la troisième température de décomposition thermique est égale ou supérieure à 150°C et égale ou inférieure à 350°C.

8. Procédé de fabrication de butadiène selon la revendication 1, dans lequel la décomposition thermique d'un polyester contenant une unité de répétition dérivée de 1,4-butanediol ou d'un mélange de polymères contenant le polyester pour fabriquer du butadiène comprend les étapes consistant à :
effectuer une première décomposition thermique du mélange de polymères comprenant un copolymère de lactide-glycolide et le polyester pour produire du lactide et du glycolide ; et
effectuer une deuxième décomposition thermique du premier mélange de polymères décomposés pour fabriquer du butadiène.

9. Procédé de fabrication de butadiène selon la revendication 8, dans lequel
la première décomposition thermique est effectuée à une température égale ou supérieure à 200°C et égale ou inférieure à 380°C, et
la deuxième décomposition thermique est effectuée à une température d'au moins 400°C,
une différence entre la première température de décomposition thermique et la deuxième température de décomposition thermique est égale ou supérieure à 100°C et égale ou inférieure à 400°C.

10. Procédé de fabrication de butadiène selon la revendication 1, dans lequel
la décomposition thermique d'un polyester contenant une unité de répétition dérivée de 1,4-butanediol ou d'un mélange de polymères contenant le polyester pour fabriquer du butadiène comprend les étapes consistant à :
effectuer une première décomposition thermique du mélange de polymères comprenant un copolymère de glycolide-3-hydroxypropionate et le polyester pour produire du glycolide ;
effectuer une deuxième décomposition thermique du premier mélange de polymères décomposés pour produire de l'acide acrylique ; et
effectuer une troisième décomposition thermique du deuxième mélange de polymères décomposés pour fabriquer du butadiène.

11. Procédé de fabrication de butadiène selon la revendication 10, dans lequel
la première décomposition thermique est effectuée à une température égale ou supérieure à 200°C et égale ou inférieure à 250°C,
la deuxième décomposition thermique est effectuée à une température égale ou supérieure à 260°C et égale ou inférieure à 380°C, et
la troisième décomposition thermique est effectuée à une température d'au moins 400°C.

12. Procédé de fabrication de butadiène selon la revendication 1, dans lequel
la décomposition thermique d'un polyester contenant une unité de répétition dérivée de 1,4-butanediol ou d'un mélange de polymères contenant le polyester pour fabriquer du butadiène comprend les étapes consistant à :
effectuer une première décomposition thermique du polyester pour produire 1,4-butanediol ; et
effectuer une deuxième décomposition thermique du premier polyester décomposé pour fabriquer du butadiène.

13. Procédé de fabrication de butadiène selon la revendication 12, dans lequel :
la première décomposition thermique est effectuée à une température égale ou supérieure à 220°C et égale ou inférieure à 350°C, et
la deuxième décomposition thermique est effectuée à une température d'au moins 400°C,
une différence entre la première température de décomposition thermique et la deuxième température de décomposition thermique est égale ou supérieure à 100°C et égale ou inférieure à 300°C.
